# EUROPEAN PATENT SPECIFICATION

(11) **EP 2 708 234 B1**
(45) Date of publication and mention of the grant of the patent: **25.05.2016**
(21) Application number: 12184067.2
(22) Date of filing: 12.09.2012
(51) Int. Cl.: A61K 38/17, C07K 14/705, C07K 16/18, C07K 16/28, A01K 67/027, G01N 33/50, A61P 37/06

(54) **Tetraspanin CD82 as a diagnostic and/or therapeutic module for xenograft recognition and/or rejection**
Tetraspanin CD82 als Diagnose- und/oder Therapiemodul zur Erkennung und/oder Abweisung eines Xenotransplantats
Tétraspanine CD82 en tant que module de diagnostic et/ou thérapeutique pour la reconnaissance et/ou le rejet de xénogreffe

(43) Date of publication of application: 19.03.2014
(73) Proprietor: King Faisal Specialist Hospital And Research Centre, Riyadh 11211 (SA)
(72) Inventor: Al-Mohanna, Futwan, Riyadh 11211 (SA); Saleh, Soad, Riyadh 11211 (SA); Parhar, Ranjit, Riyadh 11211 (SA); Al-Hejailan, Reem, Riyadh 11211 (SA); Bekheet, Razan, Riyadh 11211 (SA); Collison, Kate, Riyadh 11211 (SA)
(74) Representative: Engelhard, Markus

(56) References cited:
- WO-A1-96/34117
- -A-
- -A-
- TSAI YIEN CHE ET AL: "Dissecting the diverse functions of the metastasis suppressor CD82/KAI1", FEBS LETTERS, vol. 585, no. 20, October 2011 (2011-10), pages 3166-3173, XP002689076,
- AL-MOHANNA F ET AL: "Human neutrophil gene expression profiling following xenogeneic encounter with porcine aortic endothelial cells: the occult role of neutrophils in xenograft rejection revealed", JOURNAL OF LEUKOCYTE BIOLOGY, vol. 78, no. 1, July 2005 (2005-07), pages 51-61, XP002689077, ISSN: 0741-5400
- LI SHENGQIAO ET AL: "Xenotransplantation: role of natural immunity.", TRANSPLANT IMMUNOLOGY JUN 2009, vol. 21, no. 2, June 2009 (2009-06), pages 70-74, XP002689078, ISSN: 1878-5492
- DATABASE BIOSIS [Online] BIOSCIENCES INFORMATION SERVICE, PHILADELPHIA, PA, US; March 2004 (2004-03), CHEN Z ET AL: "CD82-siRNA down-regulates CD82 expression in thyroid carcinoma cell line FTC-133.", Database accession no. PREV200700277957
- YANG JIAN-MIN ET AL: "KAI1 gene suppresses invasion and metastasis of hepatocellular carcinoma MHCC97-H cells in vitro and in animal models", LIVER INTERNATIONAL, vol. 28, no. 1, January 2008 (2008-01), pages 132-139, ISSN: 1478-3223(print)

## Description

The present invention relates to CD82 polypeptides of the mammalian tetraspanin CD82 protein family for use in the diagnosis of xenograft recognition and/or rejection. The present invention further relates to inhibitors of CD82 polypeptides for use in the prevention and/or treatment of xenograft recognition and/or rejection. The present invention furthermore relates to CD82 knockout and transgenic animals and their cells, tissues and organs. The present invention furthermore relates to pharmaceutical compositions comprising cells, tissues and organs of animals in which the CD82 level, expression and/or activity is modified, and their use in the diagnosis, prevention and/or treatment of xenograft recognition and/or rejection.

### BACKGROUND OF THE INVENTION

After all pharmacological interventions have failed, there exists a growing number of patients requiring immediate alternatives to human organ donations; since the number of available donor organs cannot keep up with the demand for such organs. Tragically, the acute shortage of donor organs leads to so many deaths of patients in dire need of transplantation. The number of heart transplants fluctuates around the 3,700 mark as reported by the registry of the International Society for Heart and Lung Transplantation [1]. It is estimated that the number of patients requiring transplantation is around 800,000 while the total number of heart transplantation in 2007 reached a maximum of only 3,500 transplants [2].

One viable option for overcoming the donor organ shortage is the use of animal organs as replacement i.e. "xenotransplantation". Initially, a transplanted organ between discordant species appears viable and healthy, but this is rapidly followed by an acute irreversible rejection: the hyperacute rejection (HAR). HAR is attributed to xenoreactive natural antibodies (XNA) and complement activation [3-6]. XNA target galactose α1,3-galactose (Galα1,3-gal) structures that decorate proteins and lipids of the transplanted organ endothelium [7]. These "decorations" are brought about by the enzyme alpha 1,3-galactosyltransferase which is expressed in all mammals except human, apes and old world monkeys [8-10]. Many strategies have been employed in order to overcome HAR. These include, removal of the anti-Gala1,3-gal antibodies, accommodation, transgenesis and siRNA silencing of the alpha 1,3galactosyl transferase. Transgenesis gave a glimpse of hope through extending the life of the transplanted organ which, however, eventually succumbed to rejection albeit at a considerably later time [11,12]. Clinical xenotransplantation is controversial due to the identified rejection problems and the possibility of xenozoonotic diseases [10].

Previously, the inventors have identified innate immune cells as an independent player in the xenograft rejection in the absence of xenoreative natural antibodies and complement [13-15]. The inventors demonstrated that human naive neutrophils are capable of recognizing and activating porcine naive endothelial cells through a calcium dependent mechanism independently of XNA and complement and under conditions in which all binding sites for α-gal epitope are blocked by saturating concentrations of anti-α-gal antibodies [16,17]. Similar results were obtained for other innate immune cells; namely NK cells under static and flow conditions [13]. The molecular mechanism(s) underlying such recognition have yet to be determined.

There is a need for means and methods for the diagnosis, prevention and/or treatment of xenograft recognition and/or rejection.

### SUMMARY OF THE INVENTION

The objects are solved according to the invention as it is claimed in the claims.

According to the present invention this object is solved by a CD82 polypeptide of the mammalian tetraspanin CD82 protein family for use in the diagnosis of xenograft recognition and/or rejection.

According to the present invention this object is solved by an inhibitor of the CD82 polypeptide for use in the prevention and/or treatment of xenograft recognition and/or rejection, wherein said prevention and/or treatment comprises inhibiting CD82 expression and/or activity,
wherein said inhibitor is
(i) an anti-CD82 antibody against the CD82 polypeptide,
(ii) small molecule inhibitor(s) of the CD82 expression.

According to the present invention this object is solved by a knockout non-human mammal whose genome comprises a homozygous or heterozygous disruption in a gene encoding a CD82 polypeptide of the mammalian tetraspanin CD82 protein family.

According to the present invention this object is solved by a transgenic, non-human mammal, wherein the cells of said non-human mammal fail to express a functional CD82 polypeptide of the mammalian tetraspanin CD82 protein family or wherein the cells of said non-human mammal comprise a coding region of a CD82. polypeptide of the mammalian tetraspanin CD82 protein family under the control of a heterologous promoter active in the cells of said non-human mammal.

According to the present invention this object is solved by a cell, a tissue or an organ obtained from a knockout or transgenic mammal of the invention.

According to the present invention this object is solved by providing said cell(s), tissue(s) and/or organ(s) for use in the prevention and/or treatment of xenograft recognition and/or rejection.

According to the present invention this object is solved by providing said cell(s), tissue(s) and/or organ(s) for use in xenotransplantation, for example, as xenografts.

According to the present invention this object is solved by a pharmaceutical composition comprising cell(s), tissue(s) and/or organ(s) obtained from an animal in which the CD82 level, expression and/or activity is modified or inhibited, optionally, a pharmaceutical excipient, and optionally, a pharmaceutical carrier,
wherein said animal in which the CD82 level, expression and/or activity is modified or inhibited is an animal of the present invention,
and/or wherein the cell(s), tissue(s) and/or organ(s) are the cell(s), tissue(s) and/or organ(s) of the present invention.

According to the present invention this object is solved by an antibody against the CD82 polypeptide or the pharmaceutical composition according to the invention for use in the diagnosis, prevention and/or treatment of xenograft recognition and/or rejection.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

### CD82 as diagnostic and therapeutic tool

As described above, the present invention provides a CD82 polypeptide of the mammalian tetraspanin CD82 protein family for use in the diagnosis of xenograft recognition and/or rejection.

The present invention provides the use of a CD82 polypeptide of the mammalian tetraspanin CD82 protein family for the diagnosis of xenograft recognition and/or rejection.

As used herein, the term "xenotransplantation" refers the transplantation of living cells, tissues or organs from one species to another. Such cells, tissues or organs are called "xenografts," or "xenotransplants". As used herein, the term "xenotransplantation" preferably refers to the transplantation of animal living cells, tissues or organs to a patient, i.e. a human recipient.

To date no xenotransplantation trials have been entirely successful due to the many obstacles arising from the response of the recipient's immune system. This response, which is generally more extreme than in allotransplantations, ultimately results in rejection of the xenograft, and can in some cases result in the immediate death of the recipient. There are several types of rejection of xenografts, these include: hyperacute rejection (HAR), acute vascular rejection, cellular rejection, chronic rejection.

As used herein, the term "xenograft recognition and/or rejection" refers to all mechanisms of the recipient after xenotransplantation, including the above.

CD82, also known as C33 antigen or KAII originally identified as a marker for "activation/differentiation" of mononuclear cells [25], is a member of the tetraspanin family of proteins responsible for divergent cellular activities including activation, differentiation, motility, adhesion, signaling, fusion and metastasis. They are highly conserved and can be found in species as disparate as fungi and mammals. In human, CD82 is expressed in many cell types including lymphocytes, granulocytes, epithelial cells, platelets, endothelial cells and fibroblasts. Thirty four (34) mammalian tetraspanins were identified with thirty three (33) expressed in human [26]. All have four transmembrane domains with cytosolic N-and C-terminal regions and two extracellular domains with conserved CCG motif [26-28]. CD82 exists as two isoforms resulting from two distinct splice variants. CD82 is heavily palmitoylated and glycated [29,30] and together with other proteins, constitutes the tetraspanin web. The web is a complex entity invoking functional diversity in stimulus response coupling [see e.g. Lazo PA, http://atlasgeneticsoncology.org//Deep/TetraspaninID20062. html.].

The amino acid sequence of SEQ ID NO. 1 shows the human CD82 splice variant VI (Genebank Accession No. NM 002231) (isoform 1); and the amino acid sequence of SEQ ID NO. 2 shows the human CD82 splice variant V2 (Genebank Accession No. NM_001024844) (isoform 2).

In a preferred embodiment, the CD82 polypeptide comprises the amino acid sequence of SEQ ID NO. 1 or SEQ ID NO. 2 or an amino acid sequence having at least 80 % sequence identity to SEQ ID NO. 1 or 2, preferably at least 90 %, more preferably at least 95 % or at least 99 % identity.

In one embodiment, the CD82 polypeptide consists of the amino acid sequence of SEQ ID NO. 1 or SEQ ID NO. 2.

Preferably the CD82 polypeptide is encoded by a nucleotide sequence encoding the amino acid sequence of SEQ ID NO. 1 or of SEQ ID NO. 2 or an amino acid sequence having at least 80 % sequence identity to SEQ ID NO. 1 or 2, preferably at least 90 %, more preferably at least 95 % or at least 99 % identity.

In a preferred embodiment, the diagnosis of said xenograft recognition and/or rejection comprises determining CD82 expression levels in patient specimen and/or specimen of the xenograft recipient. This expression levels may be determined by mRNA levels and protein levels in cells, tissues, organs and sera and other bodily fluids.

As described above, the present invention provides an inhibitor of the CD82 polypeptide for use in the prevention and/or treatment of xenograft recognition and/or rejection.

Thereby said prevention and/or treatment of said xenograft recognition and/or rejection comprises inhibiting CD82 expression and/or activity.

The CD82 expression and/or activity can inhibited in the donor of the xenograft (such as a pig or sheep) and/or in the recipient of the xenograft (such as a patient, a human recipient).

According to the invention, the inhibitor is
(i) an anti-CD82 antibody,
(ii) small molecule inhibitor(s) of the CD82 expression.

According to the invention, an anti-CD82 antibody is an antibody against:
- the CD82 polypeptide comprising or consisting of the amino acid sequence of SEQ ID NO. 1 or SEQ ID NO. 2
   or
- the CD82 polypeptide comprising an amino acid sequence having at least 80 % sequence identity to SEQ ID NO. 1 or 2, preferably at least 90 %, more preferably at least 95 % or at least 99 % identity;
   or
- the CD82 polypeptide encoded by a nucleotide sequence encoding the amino acid sequence of SEQ ID NO. 1 or of SEQ ID NO. 2 or
- the CD82 polypeptide encoded by a nucleotide sequence encoding an amino acid sequence having at least 80 % sequence identity to SEQ ID NO. 1 or 2, preferably at least 90 %, more preferably at least 95 % or at least 99 % identity.

Preferably, small molecule inhibitor(s) of the CD82 expression are siRNA(s), antisense oligonucleotide(s), transcription and/or translation inhibitor(s).

Preferably, the inhibitor is administered to a subject in need thereof, by inhalation, intranasal, intravenous, oral, transdermal, sustained release, controlled release, delayed release, suppository, or sublingual administration.

In one embodiment, the inhibitor is administered to a subject in need thereof in combination with at least one immunosuppressive agent.

Preferably, the immunosuppressive agent is selected from azathioprene, cyclosporine, glucocorticoid and pharmaceutically acceptable salts thereof.

### CD82 knockout and transgenic animals and their cells, tissues and organs

As described above, the present invention provides CD82 knockout animals and CD82 transgenic, animals.

A CD82 knockout animal according to the invention is a knockout non-human mammal whose genome comprises a homozygous or heterozygous disruption in a gene encoding a CD82 polypeptide of the mammalian tetraspanin CD82 protein family.

The CD82 transgenic animals of the invention are animals in which the CD82 level, expression and/or activity is modified or inhibited

A CD82 transgenic animal according to the invention is a transgenic, non-human mammal, wherein the cells of said non-human mammal fail to express a functional CD82 polypeptide of the mammalian tetraspanin CD82 protein family or wherein the cells of said non-human mammal comprise a coding region of a CD82 polypeptide of the mammalian tetraspanin CD82 protein family under the control of a heterologous promoter active in the cells of said non-human mammal.

The knockout or transgenic mammal of the invention is preferably a pig or a sheep.

The CD82 of a knockout or transgenic mammal of the invention preferably refers to a CD82 polypeptide which comprises the amino acid sequence of SEQ ID NO. 1 or 2 or an amino acid sequence having at least 80 % sequence identity to SEQ ID NO. 1 or 2, preferably at least 90 %, more preferably at least 95 % or at least 99 % identity;
and/or to a CD82 polypeptide which is encoded by a nucleotide sequence encoding the amino acid sequence of SEQ ID NO. 1 or 2 or encoding an amino acid sequence having at least 80 % sequence identity to SEQ ID NO. 1 or 2, preferably at least 90 %, more preferably at least 95 % or at least 99 % identity.

In one embodiment, the CD82 of a knockout or transgenic mammal of the invention refers to a CD82 polypeptide which consists of the amino acid sequence of SEQ ID NO. 1 or of SEQ ID NO. 2.

Preferably, the CD82 knockout animals and/or the CD82 transgenic animals are used as the donor animals for xenotransplantation, i.e. as the donor animals of a xenograft.

As described above, the present invention provides cell(s), tissue(s) and/or organ(s) obtained from the CD82 knockout or transgenic mammal(s) of the invention.

As described above, the present invention provides the cell(s), tissue(s) and/or organ(s) obtained from the CD82 knockout or transgenic mammal(s) of the invention for use in the prevention and/or treatment of xenograft recognition and/or rejection.

As described above, the present invention also provides the cell(s), tissue(s) and/or organ(s) obtained from the CD82 knockout or transgenic mammal(s) of the invention for use in xenotransplantation, i.e. as xenografts.

Said use comprises the administration of the cell(s), tissue(s) and/or organ(s) of the invention to a subject in need thereof.

### Antibodies and pharmaceutical compositions and their uses

The present invention describes an antibody against a CD82 polypeptide.

For example, an antibody can be an antibody against:
- the CD82 polypeptide comprising or consisting of the amino acid sequence of SEQ ID NO. 1 or SEQ ID NO. 2
   or
- the CD82 polypeptide comprising an amino acid sequence having at least 80 % sequence identity to SEQ ID NO. 1 or 2, preferably at least 90 %, more preferably at least 95 % or at least 99 % identity;
   or
- the CD82 polypeptide encoded by a nucleotide sequence encoding the amino acid sequence of SEQ ID NO. 1 or of SEQ ID NO. 2
   or
- the CD82 polypeptide encoded by a nucleotide sequence encoding an amino acid sequence having at least 80 % sequence identity to SEQ ID NO. 1 or 2, preferably at least 90 %, more preferably at least 95 % or at least 99 % identity.

As described above, the present invention provides pharmaceutical compositions.

In one embodiment, the pharmaceutical composition comprises
cell(s), tissue(s) and/or organ(s) obtained from an animal in which the CD82 level,
expression and/or activity is modified or inhibited,
optionally, a pharmaceutical excipient, and
optionally, a pharmaceutical carrier.

According to the invention, the animal in which the CD82 level, expression and/or activity is modified or inhibited and from which the cell(s), tissue(s) and/or organ(s) are obtained from, is an CD82 knockout or transgenic mammal of the invention.

According to the invention, the cell(s), tissue(s) and/or organ(s) are the cell(s), tissue(s) and/or organ(s) obtained from the CD82 knockout or transgenic mammal(s) of the invention.

In one embodiment, the carrier, if present, is aqueous.

In one embodiment, a pharmaceutical composition of the present invention furthermore comprises at least one immunosuppressive agent.

The immunosuppressive agent is preferably selected from azathioprene, cyclosporine, glucocorticoid and pharmaceutically acceptable salts thereof.

As described above, the present invention provides an antibody against the CD82 polypeptide as defined herein or the pharmaceutical composition according to the present invention for use in the diagnosis, prevention and/or treatment of xenograft recognition and/or rejection.

As described above, the present invention provides the use of an antibody against the CD82 polypeptide as defined herein or the use of a pharmaceutical composition according to the present invention for the diagnosis, prevention and/or treatment of xenograft recognition and/or rejection.

Preferably, the antibody or the pharmaceutical composition is administered to a subject in need thereof.

The administration can be by inhalation, intranasal, intravenous, oral, transdermal, sustained release, controlled release, delayed release, suppository, or sublingual administration.

In one embodiment, the antibody or the pharmaceutical composition is administered to a subject in need thereof in combination with at least one immunosuppressive agent.

The immunosuppressive agent is preferably selected from azathioprene, cyclosporine, glucocorticoid and pharmaceutically acceptable salts thereof.

### Diagnosis methods

The present invention describes a method for the diagnosis of xenograft recognition and/or rejection.

Said method comprises determining CD82 expression levels in patient specimen.

The patient specimen can be specimen of the xenograft recipient, such as blood, serum, urine, tissue samples, cells or organs.

### Methods for preventing and/or treating xenograft recognition and/or rejection

The present invention describes method(s) for the prevention and/or treatment of xenograft recognition and/or rejection.

Said method for the prevention and/or treatment of xenograft recognition and/or rejection, comprises the step of
administering to a patient an effective amount of at least one inhibitor of a CD82 polypeptide of the mammalian tetraspanin CD82 protein family;
and/or
administering to a patient cell(s), tissue(s) and/or organ(s) obtained from an animal in which the CD82 level, expression and/or activity is modified or inhibited.

The present invention describes method(s) for the prevention and/or treatment of xenograft recognition and/or rejection through methods of xenotransplantation of a CD82-modified xenograft.

The CD82 polypeptide can comprises the amino acid sequence of SEQ ID NO. 1 or SEQ ID NO. 2 or an amino acid sequence having at least 80 % sequence identity to SEQ ID NO. 1 or 2.

The CD82 polypeptide can consist of the amino acid sequence of SEQ ID NO. 1 or SEQ ID NO. 2.

The CD82 polypeptide can be encoded by a nucleotide sequence encoding the amino acid sequence of SEQ ID NO. 1 or 2 or an amino acid sequence having at least 80 % sequence identity to SEQ ID NO. 1 or 2.

Said inhibitor can be
(i) an anti-CD82 antibody,
   such as an antibody against the CD82 polypeptide,
(ii) small molecule inhibitor(s) of the CD82 expression,
such as siRNA(s), antisense oligonucleotide(s), transcription and/or translation inhibitor(s).

The inhibitor can be administered to the patient.

The administration can be by inhalation, intranasal, intravenous, oral, transdermal, sustained release, controlled release, delayed release, suppository, or sublingual administration.

The inhibitor can be administered to the patient in combination with at least one immunosuppressive agent.

The immunosuppressive agent can be azathioprene, cyclosporine, glucocorticoid and pharmaceutically acceptable salts thereof.

Said animal in which the CD82 level, expression and/or activity is modified or inhibited can be a CD82 knockout animal or a CD82 transgenic animal according to the invention.

Said cell(s), tissue(s) and/or organ(s) are the cell(s), tissue(s) and/or organ(s) can be obtained from the CD82 knockout animal or a CD82 transgenic animal according to the invention.

The cell(s), tissue(s) and/or organ(s) can be administered to the patient.

The administration can be by inhalation, intranasal, intravenous, oral, transdermal, sustained release, controlled release, delayed release, suppository, or sublingual administration.

The cell(s), tissue(s) and/or organ(s) can be administered to the patient in combination with at least one immunosuppressive agent.

The immunosuppressive agent can be azathioprene, cyclosporine, glucocorticoid and pharmaceutically acceptable salts thereof.

The administration of the at least one inhibitor can be carried out together with an administration of the cell(s), tissue(s) and/or organ(s).

### Methods ofxenotransplantion

The present invention describes method(s) of xenotransplantation.

Said method of xenotransplantation comprises the step of
administering to a patient cell(s), tissue(s) and/or organ(s) obtained from a donor animal in which the CD82 level, expression and/or activity is modified or inhibited.

The CD82 polypeptide can comprise the amino acid sequence of SEQ ID NO. 1 or SEQ ID NO. 2 or an amino acid sequence having at least 80 % sequence identity to SEQ ID NO. 1 or 2.

The CD82 polypeptide can consist of the amino acid sequence of SEQ ID NO. 1 or SEQ ID NO. 2.

The CD82 polypeptide is can be encoded by a nucleotide sequence encoding the amino acid sequence of SEQ ID NO. 1 or 2 or an amino acid sequence having at least 80 % sequence identity to SEQ ID NO. 1 or 2.

Said animal in which the CD82 level, expression and/or activity is modified or inhibited is a CD82 knockout animal or a CD82 transgenic animal according to the invention.

Said cell(s), tissue(s) and/or organ(s) are the cell(s), tissue(s) and/or organ(s) can be obtained from the CD82 knockout animal or a CD82 transgenic animal according to the invention.

The cell(s), tissue(s) and/or organ(s) of the CD82 knockout animal or a CD82 transgenic animals of the invention can be utilized as the xenografts.

The cell(s), tissue(s) and/or organ(s) can be administered to the patient.

The cell(s), tissue(s) and/or organ(s) can be administered to the patient in combination with at least one immunosuppressive agent.

The immunosuppressive agent can be azathioprene, cyclosporine, glucocorticoid and pharmaceutically acceptable salts thereof.

### Further description of one embodiment

Here we used porcine endothelial cells from wild type and α-gal-knockout animals to demonstrate that recognition of xenogeniec endothelial cells occurs independently of α-gal structures. We used three human derived pro-myeloid cell lines; HL60, THP-1 and KG-1 which, in their undifferentiated state do not recognize xenogeneic endothelial cells as defined by the lack of calcium transient and ROM production in response to POAECs; however, after differentiation, these cells transiently raise their intracellular calcium and increase ROM production upon exposure to POAECs. In order to identify possible α-gal-independent site(s) mediating recognition of xenogeneic endothelial cells, we used Serial Analysis of Gene Expression (SAGE) of the promyelocytic cell lines together with that of human naïve neutrophils. We created SAGE libraries of these cell lines and use them to identify SAGE transcripts before and after differentiation, and compared those to SAGE transcripts in resting human naive neutrophils. SAGE libraries of these cell lines were used to compare transcriptional activities before and after differentiation witch that of human naive neutrophils. This strategy yielded a number of transcripts that were: (1) differentially expressed in all of the differentiated vs undifferentiated cell lines; (2) constitutively expressed in human naive neutrophils. Twelve differentially expressed transcripts were identified by this approach with only six (6) displaying consistent change in all cell lines. Since our putative xeno-recognition moiety(s) should be (1) trans plasma membrane protein(s) and (2) associated with intracellular Calcium release, only one out of the six identified transcripts, belonging to the tetraspanin CD82, satisfied the above criteria and was therefore considered the likely candidate mediating the recognition of xeno-endothelial cells independently of Galα1,3-gal. his was confirmed by our finding that blocking antibodies to CD82 inhibited both the calcium rise and ROM production in human naive neutrophils upon exposure to POAECs. Thus, it appears that CD82 mediated recognition is the mechanism used by innate immune cells to identify xenogeneic endothelial cells and thus responsible for delayed xenograft rejection.

Alpha-gal 1,3 gal was identified as the major barrier to xenotransplantation of animal organs into non-human primates. Hyperacute rejection of transplanted xenogeneic organs was attributed to xenoreactive natural antibodies against Galα1,3-gal decorated proteins and lipids on the xenograft and complement activation, leading to the demise of transplanted vascularized xenograft within minutes [3-6]. Organs from Galα1,3-gal knock out animals were also rejected albeit after a relatively prolonged survival with immunosupression [11]. The fact however, remains that the necessary sustained survival of transplanted xenografts is yet to be achieved, prompting a serious search for putative mechanism(s) involved in the eventual rejection of the transplanted xenograft. In addition, human naïve innate immune cells recognize, activate and are activated by xenogeneic endothelial cells in the absence of xenoreactive natural antibodies and complement, and under conditions in which all alpha-gal binding sites were blocked by anti-gal IgG [13, 15-17].

In the present work we demonstrate that human naive neutrophils are activated by xenogeneic porcine aortic endothelial cells but not by allogeneic human aortic endothelial cells or HUVECs. This suggests that the recognition of xenogeneic endothelial cells by human naive neutrophils occurs in an Galα1,3-gal-independent manner. To identify the molecular moiety(s) involved in this recognition we used progranulocytic cell lines which in their undifferentiated state are not activated by xenogeneic endothelial cells, and only become activated after differentiation into neutrophil-like or monocyte-like cells. SAGE analysis of these cells and of resting human naïve neutrophils revealed six different transcripts that were consistently over expressed in the differentiated cell lines and thus are the likely candidates' transcripts responsible for xenorecognition and subsequent activation of these cell lines by xenogeneic endothelial cells. Out of these six, only CD82 was identified as an integral plasma membrane protein, suggesting that CD82 was the likely candidate responsible for xenoendothelial cell recognition. Three lines of evidence support such a claim:
(1) Undifferentiated cell lines HL-60, KG-1 and THP-1 expressing relatively low levels of CD 82 at both the message and protein levels do not evoke a calcium transient or ROM production in response to POAECs.
(2) Differentiated HL-60, KG-1 and THP-1 and human naïve neutrophils expressing relatively higher levels of CD82 do respond to xenogeneic POAECs.
(3) Antibodies to CD82 can inhibit both calcium transient and ROM production in response to xenogeneic insult.

CD82 also known as C33 antigen or KAII originally identified as a marker for "activation/differentiation" of mononuclear cells [25], is a member of the tetraspanin family of proteins responsible for divergent cellular activities including activation, differentiation, motility, adhesion, signaling, fusion and metastasis. They are highly conserved and can be found in species as disparate as fungi and mammals. In human, CD82 is expressed in many cell types including lymphocytes, granulocytes, epithelial cells, platelets, endothelial cells and fibroblasts. Thirty four (34) mammalian tetraspanins were identified with thirty three (33) expressed in human [26]. All have four transmembrane domains with cytosolic N-and C-terminal regions and two extracellular domains with conserved CCG motif [26-28]. CD82 exists as two isoforms resulting from two distinct splice variants. CD82 is heavily palmitoylated and glycated [29,30] and together with other proteins, constitutes the tetraspanin web. The web is a complex entity invoking functional diversity in stimulus response coupling [see e.g. Lazo PA, http://atlasgeneticsoncology.org//Deep/TetraspaninID20062. html.]. Furthermore, CD82 was demonstrated to link lipid rafts to the actin cytoskeleton and depletion of cholesterol seems to inhibit CD82 dependent responses. Tetraspanins have long been considered as membrane organizers [31,32] and are known to interact with a number of integrins, growth factors, lipid rafts, actin cytoskeleton and membrane domains; suggesting their involvement in immune responses and immune synapse [33]. Immune synapse is a major intercellular contact milieu where different proteins cluster to assemble to perform a variety of functions.

Loss of CD82 expression has been correlated with increase metastasis in colorectal cancers, squamous cell carcinoma, prostate cancer, breast cancer, and hepatocellular carcinoma [e.g. 34,35]. However, its role in primary tumor growth is not well defined [36]. Tumor metastasis suppression by CD82 is mediated through a number of molecular mechanisms including stablizing E-cadherin/beta-catenin complex formation, upregulation of Sprouty2, maturation of β-1 integrin and direct interactions with DARC-expressing endothelial cells with the subsequent inhibition of tumor cell proliferation and induction of senescence [37-40]. CD82 has been associated with suppression of tumor metastasis and this fact must be reconciled when designing strategies to reduce the CD82 levels in transplanted xenografts.

In conclusion CD82 is a valuable molecular moiety for developing targeted diagnostics and therapeutic modalities in order to extend the life of a transplanted xenograft and provide a viable alternative to the chronic shortage of suitable human organs.

The following examples and drawings illustrate the present invention without, however, limiting the same thereto.

### BRIEF DESCRIPTION OF THE DRAWINGS

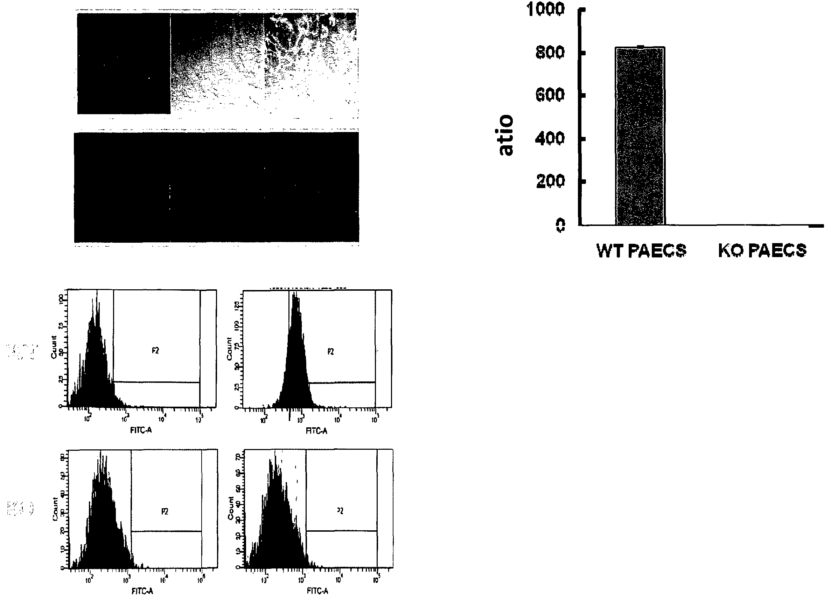

**Figure 1****:** *Expression of alpha gal in POAECs.*
   (A) Confocal fluorescence micrographs showing the expression of alpha-gal in wild type (WT) and knockout (KO) POAECs (top left), and corresponding flowcytometric histograms from WT and KO POAECs (bottom left). The message level for alpha gal transferase is shown on the top right as ratio to the house keeping protein GAPDH in WT and KO POAECs.
      *Calcium dependent recognition of xenogeneic endothelial cells by human naive neutrophils is independent of alpha-gal.*
   **(B)** Calcium changes in human neutrophils at the indicated time intervals in seconds (s) invoked by exposure to alpha gal KO POAEC (top). Calcium changes are coded such that high calcium is indicated by white. Absolute calcium levels invoked in neutrophils by alpha gal KO POAECs (bottom).
   (**C**) Calcium changes in human neutrophils invoked by WT POAECs (top left) and in the presence of saturating concentrations of antibodies to the alpha-gal (top right). Bottom graphs of (C) indicate the corresponding absolute calcium levels.

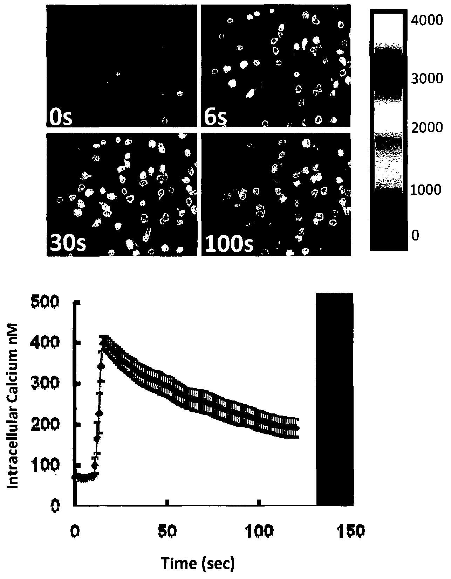

**Figure 2**: *Activation of human naïve neutrophils by xenogeneic endothelial cells but not allogeneic endothelial cells.* Reactive oxygen metabolite (ROM) production as measured by LDCL in human naive neutrophils after stimulation with POAECs (WT) and knockout POAECs (KO) and the lack of effect on HAECs.

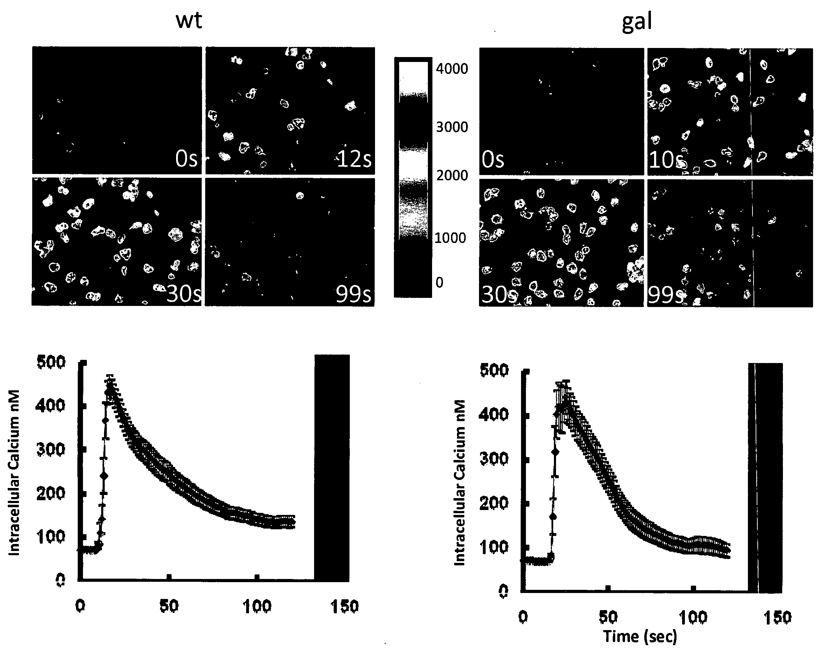

**Figure 3****:** *Undifferentiated human cell lines HL-60, THP-1 and KG-1 do not recognize xenogeneic endothelium unless differentiated into neutr-ophil-like or monocyte-like cells* NBT micrographs of undifferentiated and differentiated cell line. Differentiation is indicated by the reduction of soluble NBT to blue-black insoluble formazan (upper panel). Calcium homeostasis in undifferentiated and differentiated cell lines; KG 1, HL60 and THP-1 (lower panel). First arrow indicates the time of addition of POAECs and second arrow indicates the time of addition of ionomycin 2µM.

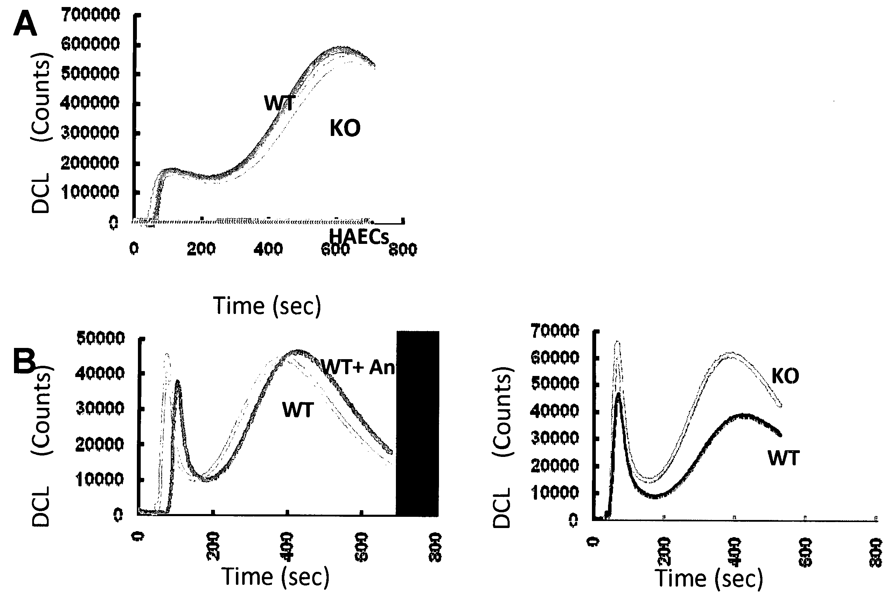

**Figure 4****:** *SAGE identifies α-gal independent xenogeneic recognition moiety(s).*
   (A) Venn diagram of the differentially expressed genes in the three cell lines upon differentiation showing a common 12 genes that are consistently and differentially expressed. The heat map identifies the differentially expressed genes and their relative expression values as indicated by the bar.
   (B) List of the differentially expressed genes in the KG-1, THP-1, HL60 cell lines expressed as fold increase and in human naive neutrophils expressed as counts.

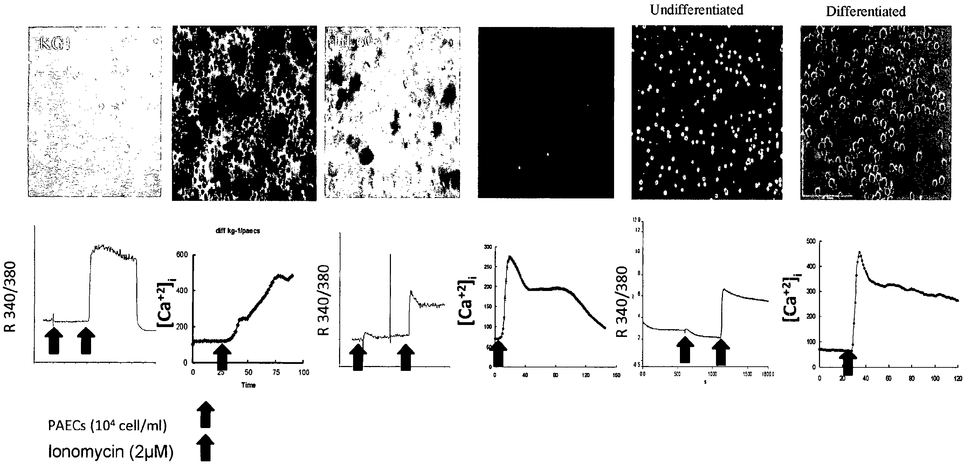

**Figure 5****:** *Expression of CD82 in differentiated and undifferentiated cell lines.*
   (A) Expression of CD 82 in differentiated and undifferentiated cell lines at the mRNA levels as indicated by ratio relative to the house keeping gene GAPDH levels.
   (B) expression of CD82 at the protein levels where N is neutrophils, HU, TU and KU are undifferentiated and HD, TD and KD are differentiated HL60, THP-1 and KG-1 respectively.
   (C) Confocal micrographs of human naive neutrophils in live cells immunostained for CD82.

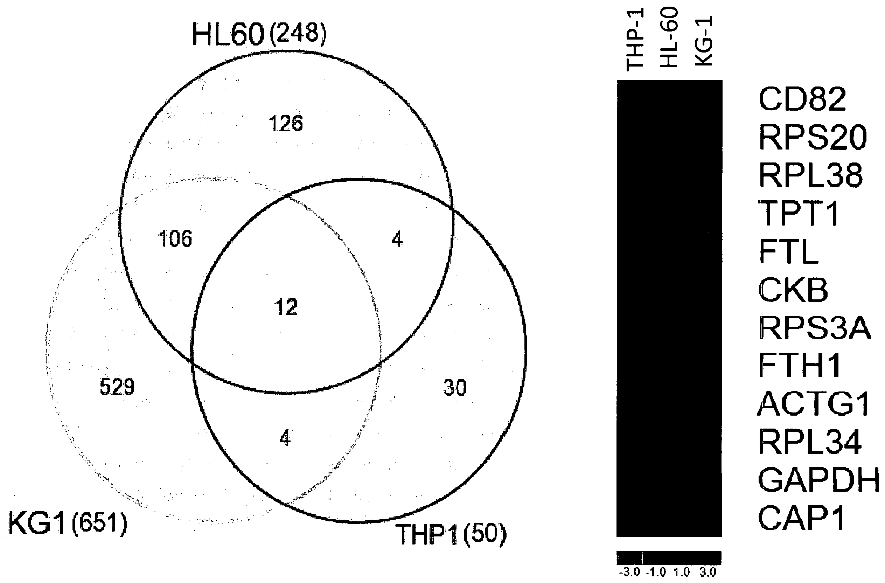

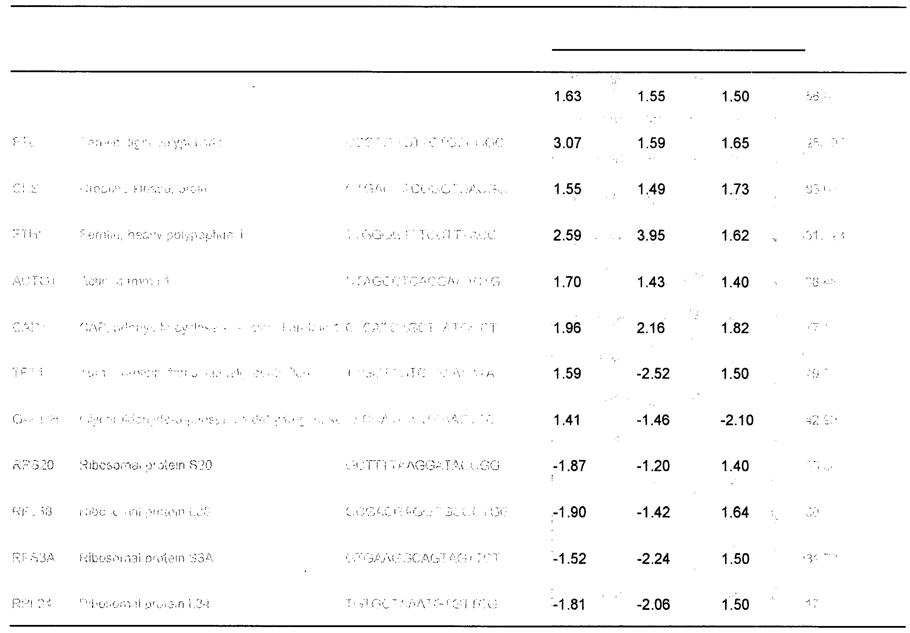

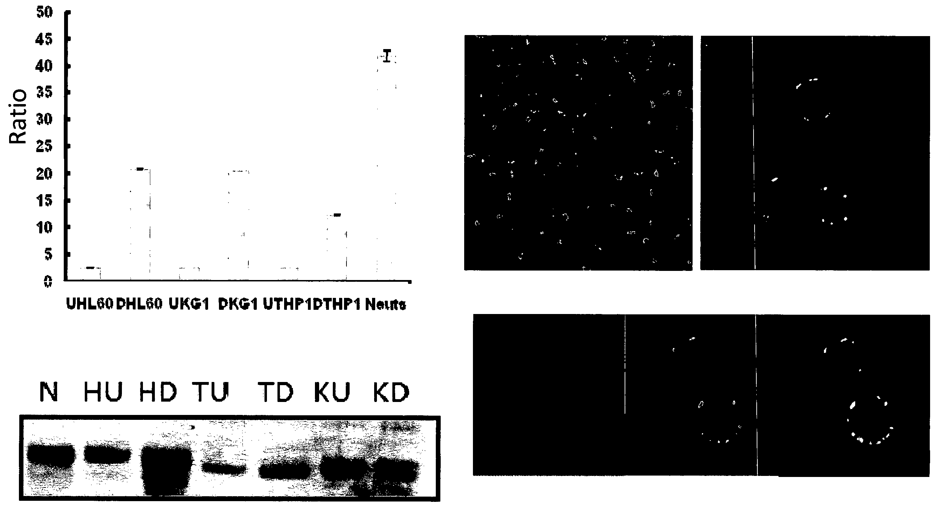

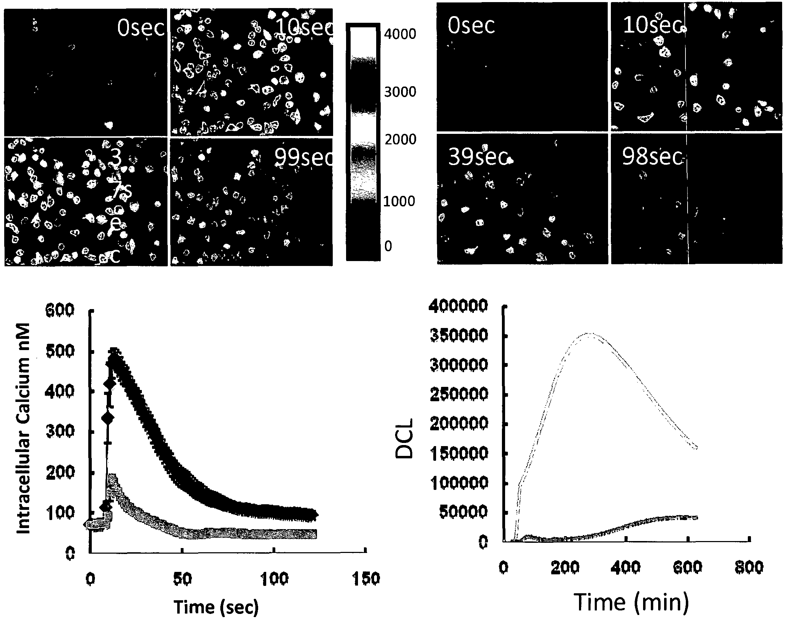

**Figure 6****:** *Inhibition of xenogeneic recognition by anti CD82 antibodies.* Inhibition of calcium dependent recognition of POAECs by human neutrophils (upper panel). The lower graphs show absolute calcium levels in human neutrophils after exposure to POAECs in the presence (squares, i.e. lower line) and absence (diamonds, i.e. upper line) of blocking antibodies to CD82 (lower left). Inhibition of ROM production as indicated by LDCL is shown in lower right in the presence (lower line) and absence (upper line) of blocking antibodies to CD82.

### EXAMPLES

### 1. Materials and Methods

### 1.1 Materials:

Fluo-3 AM (4-(6-Acetoxymethoxy-2,7-dichloro-3-oxo-9-xanthenyl)-4'-methyl-2,2'-(ethylenedioxy)dianiline-N,N,N',N'-tetraacetic acid tetrakis (acetoxymethyl) ester) was purchased from Molecular Probes (Invitrogen, Carlsbad, CA). LightCycler Instrument (Roche Diagnostics, Mannheim, Germany), LightCycler -DNA Master SYBR Green 1 was purchased from Roche Diagnostics (Mannheim, Germany). I-SAGE/I-Long SAGE kit with magnetic stand, Platinum *Taq*DNA polymerase, and Triazol solution were purchased from (Invitrogen, Carlsbad, CA). Cell lines were purchased from ATCC (ATCC, Rockville, MD). Culture media; RPMI-1640 and DMEM were purchased from (Gibco BRL, Grand Island, NY). All other reagent were Analar grade and were purchased from Sigma (MO, USA) and BDH Chemicals (UK). Fluo-3 AM, and 5-Amino-2,3-dihydro-1,4-phthalazinedione (luminol) were dissolved in dimethylsulfoxide (DMSO) and delivered to the cells at a final concentration of 1 µM, 11 µM, respectively; in a final DMSO concentration of 0.1%. Antibodies to von Willebrand Factor were purchased from (F3520; Sigma, MO); acetylated low-density lipoprotein (DiI-Ac-LDL; Biogenesis, Bournmouth UK); mouse anti human CD82 were purchsed from (Abcam and Santa Cruze, CA, USA), anti-LFA-1α were purchased from R&D System (Mineapolis, USA). Secondary goat anti mouse FITC labeled were purchased from Santa Cruze (CA, USA) and Alexa 647 labeled were from Pierce (USA). PMA and Dimethylsulphoxide (DMSO), Bt2-cAMP, 3-(4,5-dimethylthiazol-2-yl)-2,5-diphenyl tetrazolium bromide (MTT), Cell culture reagents, protease inhibitors, and other analytical grade reagents were purchased from Sigma-Aldrich (St Louis, MO). Restriction enzymes, *Nla*III, *Mme*I*,* and *Sph* were purchased from New England Biolabs Inc., (Beverly, MA).

### 1.2 Endothelial cells:

Porcine aortic endothelial cells (POAECs; P304K-05) and human aortic endothelial cells (HOAECs; 304K-05a) were purchased from Cell Application, Inc. (San Diego, Ca, USA). Human umbilical vein endothelial cells (HUVECs; CC-2517) were purchased from Lonza Group Ltd (Basel, Switzerland). POAECs and HOAECs were cultured and maintained in tissue culture medium from (Cell Application Inc. San Diego, CA. USA); whereas HUVECs were cultured and maintained in tissue culture medium purchased from (GIBCO, USA). Cells were used from passage 2-10 in all experiments at a split ratio of 1:3. To test that endothelial cells were not activated during culture, IL1- levels in conditioned medium were measured using ELISA (R&D Systems, MN.), and were consistently found to be negligible (<4 pg/ml).

### 1.3 Preparation of neutrophils:

Human peripheral blood neutrophils were prepared by dextran sedimentation of heparinized whole blood obtained from healthy donors and centrifuged through Ficoll-Paque as described previously [16]. Contaminating red blood cells were removed by hypotonic lysis with isotonic NH₄Cl. The remaining cells were suspended in Krebs-HEPES medium (pH 7.4) containing 120 mM NaCl, 1.3 mM CaCl₂, 1.2 mM MgSO₄, 4.8 mM KCl, 1.2 mM KH₂PO₄, 25 mM HEPES and 0.1 % Bovine serum albumin (BSA) and were further purified through neutrophil isolation medium (Cardinal Associates, Santa Fe, NM). Final purity and viability were both between 98-99% as indicated by flow cytometry and trypan blue dye exclusion tests. Neutrophils were routinely tested for production of reactive oxygen metabolites (ROM) by luminol-dependent chemiluminescence (LDCL) for 10 minutes. Cells were considered naive and therefore suitable for experimentation only when no increase in LDCL was observed.

### 1.4 Cell Lines: HL-60, KG-1 and THP-1:

Acute promyelocytic lukemia; HL-60 cell line (Catalog No.CCL-240), acute mylogenous Lukemia; KG-1 cell line (Catalog No.CCL-246) and acute monocytic leukemia; THP-1 cell line (Catalog No.TIB-202) were purchased from ATCC (ATCC, Rockville, MD). HL-60 and KG-1 cell lines were cultured in complete Iscoves Modified Medium (ATCC catalog No.30-2005) supplemented with 10% fetal bovine serum (ATCC catalog No. 30-2020) and penicillin (100U/ml), and streptomycin (100µg/ml). THP-1 cells were cultured in complete RPMI media (ATCC catalog No. 30-2001) supplemented with 10% Fetal bovine serum (ATCC catalog No. 30-2020) and penicillin (100U/ml), and streptomycin (100µg/ml). All cell lines were maintained in a humidified incubator at 37°C with 5% CO₂. HL-60 differentiation into neutrophil-like cells was performed by treatment of 2X10⁶ cell/ml with 1.3% DMSO (Sigma catalog No. D4540) in complete media for 6-8 days with media change every third day. Differentiation into neutrophil-like cells was ascertained by their ability to generate Reactive Oxygen Metabolite (ROM) in response to stimulation by phorbol myristate acetate; PMA (100ng/ml) or the chemotactic peptide fMLP (1 µM). This was detected by either the reduction of the soluble NBT to blue-black insoluble formazan and/or Lumiol-dependent chemilumesicence. For the former, one ml of cell suspension was incubated for 20 min at 37°C with an equal volume of 0.2% NBT (Sigma Chemical Co., St. Louis, Mo.) dissolved in phosphate-buffered saline (pH 7.2;0.15 M without Ca⁺², Mg⁺²), in the presence of 200ng of PMA. Differentiated cells contain formazan deposits as dark, irregularly shaped crystal inclusions in the cytoplasm. By Day 6, approximately 98% of the cells reduced NBT upon PMA stimulation, and less than 5% in the absence stimulation. THP-1 and KG-1 differentiation was performed as above but with treatment with Bt₂cAMP (500µg/ml) and PMA (100ng/ml) for four (4) days and five (5) days, respectively [18]. Differentiation was confirmed by ROM production as above.

### 1.5 Preparation of anti-Gal(1,3) gal antibodies:

Anti-Gal(1,3) gal antibodies were prepared essentially as described previously [19] and all proceedures approved by the Animal Care and Use Committee (IRB at KFSHRC). Briefly, 10-15-kg non human promates were immunized by intra-muscular injection of emulsified soluble Gal(1,3) gal with Hunter's TiterMax Adjuvant (Sigma, USA). The animals were given booster injections 3 weeks later. Samples of blood were taken at 5 weeks postimmunization and tested for binding to porcine thyroglobulin, soluble Gal(1,3) gal and PAECs as described previously [30]. Booster injections were given at 4-6 weeks thereafter and continued for 6-9 months. To obtain Gal(1,3) gal antiserum blood was collected in 50-ml sterile Falcon tubes and allowed to clot at room temperature for 30 min. Serum was centrifuged (3000g, 4°C, 30 min) heat-inactivated (30min, 56°C), and recentrifuged (1000g, 4°C, 30 min). Antiserum immunoglobulins (IgG, IgA and IgM) were quantified using Cobas Mira Plus System (Hoffmann-La Roche, Basel, Switzerland) before extensive dialysis against 5mM sodium phosphate buffer (pH 6.5). The dialysate was applied to equilibrated anion resin (Sephadex DEAE A-50, Pharmacia, Uppsala, Sweden) at a ratio of 2:1 (resin:supernatant). The use of this anion resin ensured that essentially all serum protein component except IgG bind to the resin, leaving an eluate rich in IgG. The eluate was fractionated by ion-exchange chromatography on Sephadex DEAE A-50. Purifies fractions were pooled, dialyzed against PBS and concentrated.

F(ab)₂ fragments of anti-Gal(1,3) gal IgG were prepared by papain digestion. Fifty microlitre (50µl) of papain solution (10mg/ml in 0.1 M sodium phosphate buffer, pH 7.0) were added to 50mg/ml of IgG in 3ml of PBS. The digestion mixture was incubated at 37°C for 4hrs. The hydrolysed product was dialyzed first against Mill-Q water then against 0.01M sodium acetate buffer (pH 5.5). Samples were removed and fractionated on a Sephacryl-S-200 column (Pharmacia). Blocking experiments with Gal(1,3) gal IgG were performed as follows: PAECs were trypsinized by incubation with Trypsin-EDTA solution for endothelial cell culture (Sigma. USA) for 2 min. at 37°C. The cells were washed three times with RPMI 1640 medium (Sigma. USA). The washed cells were then incubated with 100g/ml of the Gal(1,3) gal IgG or F(ab)₂ fragments of the antibodies at room temperature for 30 min. The suspension was then added to adherent neutrophils at a final ratio of 10:1 (neutrophils to PAECs). This concentration was previously shown to sufficiently block all Gal(1,3) gal binding sites [16].

### 1.6 Calcium measurements:

Neutrophils or appropriate differentiated cell lines were loaded with Fluo-3-AM (1µM) as described previously [17]. The cells were washed, placed on glass coverslips and allowed to adhere for 15 minutes at room temperature. Coverslips were washed then secured between two plates of a custom-designed coverslip holder, placed onto a heated microscope stage (37° C) and [Ca⁺⁺]ᵢ images were acquired at 1-2 seconds intervals. For each coverslip 100µl of POAECs , suspended in Krebs-Hepes buffer (pH 7.4), containing 10⁴ cells were added and image acquisition was continued for at least 5 minutes. Control experiments were carried out using equal number of HOAECs or HUVECs/coverslip. Images were analyzed using UltraView confocal software (PerkinElmer, UK) and fluorescence intensity (from each cell) was transformed into absolute calcium levels as described previously [1.6]. Because undifferentiated cell lines were none adherent, calcium measurements were carried out using fluorimetric assays (PerkinElmer LS 55 Luminescence Spectrometer, PerkinElmer, UK) with cells labeled with fura-2 AM as described previously [20]. Experiments were analyzed using FL WinLab software (PerkinElmer, UK).

### 1.7 Measurement of Reactive Oxygen Metabolite production:

The production of Reactive Oxygen Metabolites (ROM) by neutrophils and the three cell lines were measured using luminol-dependent chemiluminescence (LDCL) on an FB12 single tube luminometer (Berthold Detection Systems, Titertek Instrument, Inc., Huntsville, USA), as essentially described previously [15]. Briefly 1.5ml of cells (suspended in Krebs-Hepes, pH 7.4) containing 10⁶ cells were challenged with 150µl containing 10⁴ PAECs (suspended in Krebs-Hepes buffer, pH 7.4) and LDCL was followed for 15 minutes. For control experiments PAECs were replaced with HOAECs or HUVECs.

### 1.8 RNA isolation

Total cellular RNA was isolated from (1-2)×10⁷ differentiated/undifferentiated cell lines and human naïve neutrophils using tri-Reagent (MRC, Cincinati, OH) following manufacturer's instructions. RNA integrity was routinely checked using 500 ng/ml of RNA on 1% denaturing agarose gel.

### 1.9 Construction of 5' Long SAGE Libraries:

SAGE was performed according to the Serial Analysis of Gene Expression detailed Protocol Version C, and analyzed using SAGE analysis software version 4.5 (Johns Hopkins University, Baltimore, MD, USA). In brief, Ten micrograms (10µg) of total RNA was bound to solid phase oligo (dT) magnetic beads. The cDNA was synthesized directly on the oligo(dT) bead. Oligo(dT) bound to magnetic beads was used as a template for the first strand cDNA synthesis, followed by the second-strand cDNA synthesis. The captured cDNAs were then digested with an "anchoring" restriction enzyme, *Nla* III, which left a 3' overhang. Complementary cDNA synthesis and *Nla* III digestion was verified using PCR . The 3' fragments were then isolated using the magnetic beads, and equally divided into two pools and ligated to two different linkers, A or B. Both linkers contain the recognition sequence for a "tagging" restriction enzyme(type IIs restriction enzyme) *Mme.*The tagging enzyme produced a staggered cut, offset by about 17bp 3' from the recognition sequence. The two linkers were ligated onto the *Nla* III overhangs. The efficiency of ligation was assessed by PCR. Subsequent digestion with *Mme* released the adapter with a short tag of cDNA from the beads. These tags were then ligated tail-to-tail, to form 130-bp ditags. The resulting ditags were PCR amplified using primers specific to each linkers , pooled, precipitated and gel purified. The linkers were released by digesting with *Nla*III, and the resulting 34-bp ditags were gel purified, concatenated and resolved on 8% (w/v) polyacrylamide gel. The high molecular weight bands (300-500bp, 500-800bp, and 800bp-1kb) were gel purified, and cloned into Sph1-linearized pZero-1 vector (Invitrogen, Carlsbad,CA, USA). Ligation products were transformed into One Shot TOP10 electrocompetent cells. Transforments were analyzed by colony PCR. Approximately 4000-5000 clones for each library were cycle sequenced using M13 forward primer and analyzed on Applied Biosystems DNA Sequencer. Each concatamer insert results in a randomly organized "series" of ditags of approx 34 bp, each flanked by the recognition sequence of the primary anchoring enzyme *Nla*III, CATG sequence that provide a "SAGE tag" specific to each expressed gene. Approximately 20-25 individual tags were were produced per clone. SAGE software was used to convert these sequences into long SAGE tags and tabulated tag abundances. Resultant SAGE tags were analyzed using the downloadable reference sequence database SAGEmap(Lash A.E etal, 2000) from the NCBI Web site. By determining the frequency distribution of the total tag population, the statistical picture of the relative abundance of the different mRNAs expressed in the differentiated vs undifferentiated cell population was obtained. Using this method (SAGE raw 17mer data) we generated five libraries, for human naive neutrophils, differentiated and undifferentiated HL60 and KG1 Cell lines. Whereas the 10-mer tag count and differential expression results for THP-1 cells were from the GEO data repository at NCBI (accession # GSE1439). All 17- and 10 mer SAGE tags identified for the three cell lines and raw tag counts were aggregated for each gene across multiple tags. Differential expression significance for these aggregated counts was determined using a z-test, where z=(x_{d}-Xᵤ)/v(x_{d}+Xᵤ), and p-values assigned as <0.01 for z > 2.58 and <0.05 for z> 1.96, according to a univariate normal distribution. Subsequently differentially expressed genes in all three cell lines (p-value <0.05) were considered.

### 1.10 Quantification of specific transcripts with LightCycler RT-PCR.

Total RNA was prepared by the guanidine isothiocyanate method using TRI REAGENT (Sigma, USA) according to the manufacturer's instructions. The RNA concentration was measured by microspectrophotometry (NanoDrop Technologies, Wilmington, DE). cDNA was synthesized from the total RNA using AMV Reverse Transcriptase (Promega, WI, USA) according to the manufacturers protocol. cDNA synthesis were performed in a final volume of 20 µl. Briefly, 2 µl of (1:10dil) of the cDNA were used for amplification in the SYBRgreen format using the LightCycler FastStart DNA Master SYBRGreen I kit (Roche Diagnostics, catalog no. 2 239 264). Primers derived from the human gene sequence were designed using the Oligo 6 Primer analysis software (Applied Biosystems, Foster City, CA). Mastermixes for human CD82 and GAPDH mRNA, and porcine alpha-GAL transferase and GAPDH mRNA were prepared according to the manufacturer's instructions, using the following primer sets:
CD82 splice variant VI (ACCESSION NM_002231)

| | | |
|---|---|---|
| Forward | 5'-GGTCCTGTCCATCTGCTTGT-3' | [SEQ ID NO. 3] |
| Reverse | 5'-CCAGAAAGCCCCCTACTTTC-3' | [SEQ ID NO. 4] |

CD82 splice variant V2 (ACCESSION NM_001024844)

| | | |
|---|---|---|
| Forward | 5'-GATGGTCCTGTCCATCTGCT-3' | [SEQ ID NO. 5] |
| Reverse | 5'-CCAGAAAGCCCCCTACTTTC -3' | [SEQ ID NO. 6] |

human GAPDH (ACCESSION NM_002046)

| | | |
|---|---|---|
| Forward | 5'-GGTGAAGGTCGGAGTCAAC-3' | [SEQ ID NO. 7] |
| Reverse | 5'-ATGGGTGGAATCATATTGGA-3' | [SEQ ID NO. 8] |

POAECS alpha 1,3-galactosyltransferase (ACCESSION NM_L36535),

| | | |
|---|---|---|
| Forward | 5'-CAGTGGTATGGGAAGGCACT-3' | [SEQ ID NO. 9] |
| Reverse | 5'-AGATGACTTTGTGGCCAACC-3' | [SEQ ID NO. 10] |

and POAECS GAPDH (ACCESSION NM_001206359),

| | | |
|---|---|---|
| Forward | 5'-GTCGGTTGTGGATCTGACCT-3' | [SEQ ID NO. 11] |
| Reverse | 5'-AGCTTGACGAAGTGGTCGTT-3' | [SEQ ID NO. 12] |

Quantitative PCR was performed using the LightCycler 480 system (Roche Diagnostics, Mannheim, Germany). Briefly, to the 8 µl of LightCycler mastermix a maximum of 10 ng cDNA in a 2 µl volume was added as PCR template. A no-target control received 8 µl of reaction mixture with 2 µl of water. Sealed capillaries were centrifuged (5s at 1000 rpm) using the LightCycler centrifuge adapters and placed into the LightCycler rotor. PCR amplification was performed in triplicate wells. The following temperature profile was utilized for amplification: denaturation for 1 cycle at 95°C for 30 s and 40 cycles at 95°C for 10 s (temperature transition, 20°C/s), 64 to 50°C (step size, 1°C; step delay, 5 cycle) for 15 s (temperature transition, 20°C/s), and 72°C for 15 s (temperature transition,2°C/s) with fluorescence acquisition at 55 to 50°C in single mode. Melting-curve analysis was done at 45°C to 90°C (temperature transition,0.2°C/s) with continuous fluorescence acquisition. Sequence-specific standard curves were generated using 10-fold serial dilutions (10² to 10⁸ copies/µl) of known amounts of cDNA. The respective concentration for any given sample was calculated using crossing cycle analysis provided by the LightCycler software. For realistic quantifications, the start amount of RNA was the same for all samples. Minor sampling errors were avoided by normalization with the housekeeping gene G3PDH.

### 1.11 Immunofluorescence and confocal microscopy:

Immunofluorescence was performed essentially as described previously [16]. Briefly, 50µl of live cell suspension (10⁷ cells /ml) were incubated primary antibodies at 1:250 dilution for one hour on ice. Cells were washed and treated with secondary antibodies at 1:500 dilution for I hour on ice. Cells were washed and spotted on the center of a coverslip which was sandwiched between two plates of specially designed holder and viewed using Zeiss Meta 510 Confocal Microscope (Zeiss, Jena, Germany). The same antibodies were used for POAECs CD82 immunofluorescence.

### 2. Results

### 2.1 Calcium dependent recognition of xenogeneic endothelial cells by human naive neutrophils is independent of alpha-gal:

When human naive neutrophils were exposed to POAECs (2x10⁶/ml), their intracellular free calcium concentrations [Ca⁺⁺]ᵢ rose from the resting level of 70±0.1 nM to 499±33nM before decaying back to pre POAECs encounter (fig1). This rise was largely dependent upon release from intracellular store(s), since parallel experiments performed in calcium free medium in the presence of extracellular EGTA (1mM) showed no significant difference in the extent of POAECs-induced calcium rise. The calcium response was always asynchronous and heterogeneous. The calcium transient was affected by neither the presence of saturating concentration of anti-α-gal antibodies nor the absence of xenoreactive natural antibodies and complement. Neither HOAECs nor HUVECs evoked any calcium rise in human naive neutrophils.

### 2.2 Activation of human naïve neutrophils by xenogeneic endothelial cells but not allogeneic endothelial cells:

Activation of human naive neutrophils following xenogeneic encounter was tested by measuring reactive oxygen metabolite (ROM) production using Luminol-Dependent Chemilumenescence (LDCL). In a series of experiments we found that POAECs cells invoked ROM production in human naive neutrophils (fig. 2a). In contrast neither HOAECs nor HUVECs exhibited any effect(s) on ROM production (fig. 2a). Parallel experiments in the presence of saturating concentrations of antibodies to the α-gal failed to yield any statistically different effect(s) on ROM production by POAECs (fig.2b). The question therefore arises as to the identity of the α-gal-independent site(s) mediating the recognition of xenogeneic endothelial cells by innate immune cells.

### 2.3 Undifferentiated human cell lines HL-60, THP-1 and KG-1 do not recognize xenogeneic endothelium unless differentiated into neutrophil-like or monocyte-like cells:

Since both wild-type and α-gal-knockout xenogeneic endothelial cells were recognized by human naïve neutrophils and since the latter are terminally differentiated thus possess all the necessary components for such recognition, we investigated the ability of less differentiated human derived cell lines to recognize xenogeneic endothelial cells. We tested three myeloid leukemic cell lines; HL60 and THP-1 which differentiate into neutrophil-like cells, and KG-1 which differentiate into monocyte-like cells, respectively [21-24]. The undifferentiated promyeloblast cell line HL-60 assumes a nonadherent spherical morphology which changes into a neutrophil-like phenotype, with adhesive capabilities upon differentiation in 1.25% DMSO for 7 days. Using a ratiometric calcium dye Fura-2-AM labeled undifferentiated the HL-60 cell suspension, we found that POAECs were unable to evoke a significant calcium rise in HL60 cell suspension (fig. 3). In addition these undifferentiated cells failed to mount a ROM production response upon exposure to the xenogeneic endothelial cells. In contrast, differentiated HL60 cells displayed a transient calcium rise with calcium levels reaching 274 ± 3 nM after exposure to POAECs from a resting level of 70 ± 0.01 nM (fig. 3). This response was concomitant with ROM production in the differentiated cells demonstrating their activation by xenogeneic endothelial cells. Similar results were obtained with the other two cell lines; namely THP-1 which upon treatment with Bt2-cAMP differentiate into neutrophil like cells, and KG-1 which upon treatment with PMA differentiate into monocyte-like cells. In all of the three cell lines the xenogeneic recognition capabilities were evident only after differentiation.

### 2.4 The use of Serial Analysis of Gene Expression (SAGE) to identify the α-gal independent xenogeneic recognition moiety(s):

Since only differentiated cell lines and terminally differentiated neutrophils were able to recognize the xenogeneic POAECs, the possibility existed that common molecular moiety(s) in the four cell types may be responsible for this recognition. We therefore used Serial Analysis of Gene Expression (SAGE) to identify the differentially expressed transcript(s) in the three cell lines and in human naive neutrophils. We used a snap shot approach of using mRNA transcripts of undifferentiated and post differentiation of HL-60 and KG-1 cells, within which our recognition moiety(s) were expected to exist. Undifferentiated HL-60 exhibited 14578 transcripts after sequencing 20261 tags, whereas differentiated HL60 exhibited 16277 transcripts following 18206 sequenced tags (Figure 4B). Out of those transcripts, 248 significantly differentially expressed (p<0.05). Similarly undifferentiated KG-1 cells exhibited 31311 transcripts obtained from 38793 sequenced tags compared to 22084 transcripts obtained from 29810 sequenced tags in the differentiated KG-1. Out of all transcripts in KG-1 cells (undifferentiated and differentiated) 651 were significantly differentially expressed. The differentially expressed transcripts from both cell types were then compared to differentially expressed transcripts of THP-1 cell line available from public library (the GEO data repository at NCBI, Accession GSE1439). This approach identified 12 differentially and significantly expressed transcripts (p ≤0.05) common to all three cell lines since they differentiate into neutrophil-/monocyt-like cells. Six transcripts displayed levels of expression that was not consistent in all three cell lines and were therefore excluded (Figure 4A and 4B) leaving six differently expressed transcripts that were consistently up regulated in the three cell lines and in human naive neutrophils. Since our target(s) were expected to be associated with the plasma membrane, we used the Database for Annotation, Visualization and Integrated Discovery (DAVID) v6.7 (http://david.abcc.ncifcrf.gov/), GOSTAT software and Goa_human database (http://gostat.wehi.edu.au/), IPA (https:Hanalysis.ingenuity.com/pa/public/security.jsp) and Pathway Studio (http://www.ariadnegenomics.com/support/pathway-studio-8/) to analyze the cellular locations of the six transcripts identified above. Five of these transcripts, namely, ferritin light chain (FTL), ferritin heavy chain (FTH1), gamma actin (ACTG1), Creatine kinase (CKB) and adenylate cyclase-associated protein (CAP)-1, were all assigned a cytoplasmic location and were therefore excluded. This left the tetraspanin CD82 as the only differentially expressed transplasma membrane protein that is associated with the ability of differentiated cell lines and human naive neutrophils to recognize xenogeneic endothelial cells independently of the alpha-gal (fig. 4).

### 2.5 Confirmation of SAGE results at the mRNA and protein levels:

To confirm the differential expression of CD82 transcripts we used qRT-PCR and western blot analysis on samples from undifferentiated and differentiated cell lines and human naive neutrophils. We found that in the undifferentiated cells, the ratio of CD82 mRNA transcript relative to the GAPDH was 2.40 ± 0.03, and this ratio rose to 20.74 ± 0.13 upon differentiation. Similar results were obtained in the other two cell lines, namely, KG-1 and THP-1 (fig. 5a). These changes in the message levels were echoed by the increase in the respective protein levels in western blot experiments (fig. 5a). Localization of the expressed CD82 was then examined by confocal microscopy of live human naïve neutrophils using indirect immunofluorescence and found to be associated with the plasma membrane (fig.5b). Confirmation of this location was done by colocalization of CD82 with the adhesion molecule LFA-1 double label of live neutrophils with antibodies to CD82 and LFA-1 (fig.5c).

### 2.6 Inhibition of xenogeneic recognition by anti CD82 antibodies:

Since SAGE, qRT-PCR, western and Confocal data have identified CD82 as the likely candidate for xenogeneic recognition, we argued that blocking CD82 by anti CD82 antibodies should inhibit recognition of POAECs by human naïve neutrophils. In a series of experiments we have exposed the latter cell type to anti-CD 82 antibodies (1µg/ml for 15 minutes at RT) prior to xenogeneic contact. We found that treatment with anti CD82 antibodies significantly (*p<0.0001)* reduced POAECs- induced calcium rise in human naive neutrophils from 482±24 nM to 183±12 nM (fig.6). Concomitantly, activation of human naïve neutrophils was significantly inhibited (fig.6).

The features disclosed in the foregoing description, in the claims and/or in the accompanying drawings may, both separately and in any combination thereof, be material for realizing the invention in diverse forms thereof.

### REFERENCES

1. Stehlik J, E.L., Kucheryavaya AY, Benden C, Christie JD, Dobbels F, Kirk R, Rahmel AO, Hertz MI. and A. Sourcelnternational Society for Heart and Lung Transplantation, Texas, USA., The Registry of the International Society for Heart and Lung Transplantation: Twenty-eighth Adult Heart Transplant Report--2011. J Heart Lung Transplant. 2011. 30(10): p. 1078-94.
2. Korfer, R., The Heart-Makers: The Future of Transplant Medicine. (documentary film). Germany: LOOKS film and television., 2007.
3. Cooper DKC, Depletion of natural antibodies in non-human primates-α step towards successful discordant xenografting in man. Clin Transplant, 1992;. 6:
4. Dalmasso, A.P., et al., Mechanism of complement activation in the hyperacute rejection of porcine organs transplanted into primate recipients. Am, J. Pathol, 1992 140(5):(0002-9440 (Print)): p. 1157-66.
5. Saadi, S. and J.L. Platt, Transient perturbation of endothelial integrity induced by natural antibodies and complement. J Exp Med, 1995. 181(1)(0022-1007 (Print)): p. 21-31.
6. Fryer, J., et al., Beyond hyperacute rejection. Accelerated rejection in a discordant xenograft model by adoptive transfer of specific cell subsets. Transplantation,, 1995. 59(2)(0041-1337 (Print)): p. 171-6.
7. Good AH, Cooper DKC, and M.A.e. al., Identification of carbohydrate structures that bind human antiporcine antibodies: implications for discordant xenografting in man. Transplant Proc. , 1992;. 24**:.**
8. Galili, U., Rachmilewitz, E.A., Peleg, A., and Flechner, I., A unique natural human IgG antibody with anti-a-galactosyl specificity.. J. Exp. Med. , 1984. 160:: p. 1519-1531.
9. Galili, U., The alpha-gal epitope and the anti-Gal antibody in xenotransplantation and in cancer immunotherapy. Immunol Cell Biol. 2005 Dec;:, 2005. 83((6) 0818-9641 (Print)): p. 674-86.
10. Byrne GW and McGregor CGA, Cardiac xenotransplantation: progress and challenges. Curr Opin Organ Transplant, 2012. 17: p. 148-154.
11. Kazuhiko Yamada, K.Y., Akira Shimizu, Takehiro Iwanaga, Yosuke Hisashi, Matthew Nuhn, Patricia O'Malley, Shuji Nobori, Parsia A Vagefi, Clive Patience, Jay Fishman, David K C Cooper, Robert J Hawley, Julia Greenstein, Henk-Jan Schuurman, Michel Awwad, Megan Sykes & David H Sachs, Marked prolongation of porcine venal xenograft survival in baboons through the use of α1,3-galactosyltransferase gene-knockout donors and the cotransplantation of vascularized thymic tissue. Nature Medicine (2005) 11, : p. 32 - 34.
12. Kenji Kuwaki, Y.-L.T., Frank J M F Dor, Akira Shimizu, Stuart L Houser, Todd M Sanderson, Courtney J Lancos, Derek D Prabharasuth, Jane Cheng, Kathleen Moran, Yosuke Hisashi, Nicolas Mueller, Kazuhiko Yamada, Julia L Greenstein, Robert J Hawley, Clive Patience, Michel Awwad, Jay A Fishman, Simon C Robson, Henk-Jan Schuurman, David H Sachs & David K C Cooper., Heart transplantation in baboons using al, 3-galactosyltransferase gene-knockout pigs as donors: initial experience. Nature Medicine (2005) 11, : p. 29 - 31
13. Sheikh S, P.R., Kwaasi A, Collison K, Yacoub M, Stern D, Al-Mohanna F., Alpha-gal-independent dual recognition and activation ofxenogeneic endothelial cells and human naïve natural killer cells. Transplantation., 2000 Sep 27; 70((6):): p. 917-28.
14. Sheikh S, P.R., Al-Mohanna F., Rapid static adhesion of human naïve neutrophil to naïve xenoendothelium under physiologic flow is independent of Galalphal, 3-gal structures. J Leukoc Biol., 2002 Jun;. 71((6):): p. 932-40.
15. Al-Mohanna F, S.S., Parhar RS, Khabar K, Collison K., Human neutrophil gene expression profiling following xenogeneic encounter with porcine aortic endothelial cells: the occult role of neutrophils in xenograft rejection revealed. J Leukoc Biol. , 2005 Jul;. 78((1):.): p. 51-61.
16. Al-Mohanna F, C.K., Parhar R, Kwaasi A, Meyer B, Saleh S, Allen S, al-Sedairy S, Stern D, Yacoub M., Activation of naive xenogeneic but not allogeneic endothelial cells by human naive neutrophils: a potential occult barrier to xenotransplantation. Am J Pathol. , 1997 Jul;. 151((1):): p. 111-20.
17. Cardozo LA, R.D., Ambrose LR, Midulla M, Florey O, Haskard DO, Warrens AN., The neutrophil: the unnoticed threat in xenotransplantation? Transplantation. , 2004 Dec 27;. 78((12):): p. 1721-8.
18. Ishibashi K, O.S., Hiramatsu M., Simultaneous measurement of superoxide generation and intracellular Ca2+ concentration reveals the effect of extracellular Ca2+ on rapid and transient contents of superoxide generation in differentiated THP-1 cells. Biochem Biophys Res Commun., 2006 Jura 2;. 344((2):): p. 571-80. Epub 2006 Mar 10.
19. Maruyama S, C.E.r., DeMartino C, Wang CY, Chen J, Al-Mohanna F, Nakeeb SM, D'Agati V, Pernis B, Galili U, Godman G, Stern DM, Andres G., Interaction of baboon anti-alpha-galactosyl antibody with pig tissues. Am J Pathol. 1999 Nov;. 155((5):):p. 1635-49.
20. Al-Mohanna FA, H.M., The use of fura-2 to determine the relationship between cytoplasmic free Ca2+ and oxidase activation in rat neutrophils. Cell Calcium. , 1988 Feb; 9(1):17-26.
21. Collins SJ, R.F., Gallagher RE, Gallo RC., Normal functional characteristics of cultured human promyelocytic leukemia cells (HL-60) ofter induction of differentiation by dimethylsulfoxide. J Exp Med. , 1979 Apr 1;. 149((4):): p. 969-74.
22. Thompson BY, S.G., Britigan BE, Rosen GM, Cohen MS., Oxygen metabolism of the HL-60 cell line: comparison of the effects of monocytoid and neutrophilic differentiation. J Leukoc Biol., 1988 Feb;. 43((2):): p. 140-7.
23. Momose H, K.H., Tsujimoto N, Kontani K, Tsujita K, Nishina H, Katada T., Dual phosphorylation ofphosphoinositide 3-kinase adaptor Grb2-associated binder 2 is responsible for superoxide formation synergistically stimulated by Fc gamma and formyl-methionyl-leucyl-phenylalanine receptors in differentiated THP-1 cells. J Immunol. , 2003 Oct 15;. 171((8):): p. 4227-34.
24. Koeffler HP, B.-E.M., Territo M., Phorbol diester-induced macrophage differentiation of leukemic blasts from patients with human myelogenous leukemia. J Clin Invest. , 1980 Nov;. 66((5):): p. 1101-8.
25. Lebel-Binay S, L.C., Fradelizi D, Conjeaud H., CD82, tetra-span-transmembrane protein, is a regulated transducing molecule on U937 monocytic cell line. J Leukoc Biol. 1995 Jun;. 57((6):): p. 956-63.
26. Huang S, Y.S., Dong M, Su J, Yu C, Shen Y, Xie X, Yu Y, Yu X, Chen S, Zhang S, Pontarotti P, Xu A., The phylogenetic analysis of tetraspanins projects the evolution of cell-cell interactions from unicellular to multicellular organisms. Genomics., 2005 Dec;. 86((6):): p. 674-84. Epub 2005 Oct 20.
27. Seigneuret M, D.A., Lagaudrière-Gesbert C, Conjeaud H., Structure of the tetraspanin main extracellular domain. A partially conserved fold with a structurally variable domain insertion. J Biol Chem., 2001 Oct 26;. 276((43):): p. 40055-64. Epub 2001 Aug 1.
28. Cannon KS, C.P., Quality control of transmembrane domain assembly in the tetraspanin CD82. EMBO J., 2001 May 15;. 20.((10):): p. 2443-53.
29. Zhou B, L.L., Reddivari M, Zhang XA., The palmitoylation of metastasis suppressor KAI1/CD82 is important for its mobility- and invasiveness-inhibitory activity. Cancer Res. , 2004 Oct 15;. 64((20):): p. 7455-63.
30. Wang H, Z.W., Zhao J, Zhang L, Liu M, Yan G, Yao J, Yu H, Yang P., N-Glycosylation pattern of recombinant human CD82 (KAI1), a tumor-associated membrane protein. J Proteomics., 2012 Feb 2;. 75((4):): p. 1375-85. Epub 2011 Nov 20.
31. Delaguillaumie A, H.J., Kohanna S, Bismuth G, Rubinstein E, Seigneuret M, Conjeaud H., Tetraspanin CD82 controls the association of cholesterol-dependent microdomains with the actin cytosreletcn in T lymphocytes: relevance to co-stimulation. J Cell Sci., 2004 Oct 15;. 117((Pt 22):): p. 5269-82. Epub 2004 Sep 28.
32. Vogt AB, S.S., Kropshofer H., Clustering of MHC-peptide complexes prior to their engagement in the immunological synapse: lipid raft and tetraspan microdomains. Immunol Rev., 2002 Nov;. 189:: p. 136-51.
33. Mittelbrunn M, Y.-M.M., Sancho D, Ursa A, Sánchez-Madrid F., Cutting edge:. dynamic redistribution of tetraspanin CD81 at the central zone of the immune synapse in both T lymphocytes and APC. J Immunol., 2002 Dec 15;. 169((12):):p. 6691-5.
34. Maurer CA, G.H., Friess H, Beyermann B, Willi D, Netzer P, Zimmermann A, Büchler MW., Reduced expression of the metastasis suppressor gene KAI1 in advanced colon cancer and its metastases. Surgery. , 1999 Nov;. 126((5):): p. 869-80.
35. Yang JM, P.Z., Si SH, Liu WW, Luo YH, Ye ZY., KAI1 gene suppresses invasion and metastasis of hepatocellular carcinoma MHCC97-H cells in vitro and in animal models. Liver Int. , 2008 Jan;. 28((1):): p. 132-9. Epub 2007 Nov 19.
36. Tsai YC, W.A., Dissecting the diverse functions of the metastasis suppressor CD82/KAI1. FEBS Lett., 2011 Oct 20;. 585((20):): p. 3166-73. Epub 2011 Aug 27.
37. Abe M, S.T., Takahashi M, Ishii K, Shimoda M, Shirasuna K., A novel function of CD82/KAI-1 on E-cadherin-mediated homophilic cellular adhesion of cancer cells. Cancer Lett., 2008 Aug 8;. 266((2):): p. 163-70. Epub 2008 Apr 18.
38. Mu Z, W.H., Zhang J, Li Q, Wang L, Guo X., KAI1/CD82 suppresses hepatocyte growth factor-induced migration of hepatoma cells via upregulation of Sprouty2. Sci China C Life Sci., 2008 Jul;. 51((7):): p. 648-54. Epub 2008 Jul 13.
39. Jee BK, L.J., Lim Y, Lee KH, Jo YH., Effect of KAI1/CD82 on the beta1 integrin maturation in highly migratory carcinoma cells. Biochem Biophys Res Commun., 2007 Aug 3;. 359((3):): p. 703-8. Epub 2007 May 30.
40. Bandyopadhyay S, Z.R., Chaudhuri A, Watabe M, Pai SK, Hirota S, Hosobe S, Tsukada T, Miura K, Takano Y, Saito K, Pauza ME, Hayashi S, Wang Y, Mohinta S, Mashimo T, Iiizumi M, Furuta E, Watabe K., Interaction of KAI1 on tumor cells with DARC on vascular endothelium leads to metastasis suppression. Nat Med., 2006 Aug;. 12((8):): p. 933-8. Epub 2006 Jul 23.

## Claims

1. A CD82 polypeptide of the mammalian tetraspanin CD82 protein family for use in the diagnosis of xenograft recognition and/or rejection.

2. The CD82 polypeptide for use according to claim 1, wherein the CD82 polypeptide comprises the amino acid sequence of SEQ ID NO. 1 or 2 or an amino acid sequence having at least 80 % sequence identity to SEQ ID NO. 1 or 2,
and/or wherein the CD82 polypeptide is encoded by a nucleotide sequence encoding the amino acid sequence of SEQ ID NO. 1 or 2 or encoding an amino acid sequence having at least 80 % sequence identity to SEQ ID NO. 1 or 2.

3. The CD82 polypeptide for use according to claim 1 or 2, wherein the diagnosis of said xenograft recognition and/or rejection comprises determining CD82 expression levels in patient specimen.

4. An inhibitor of the CD82 polypeptide for use in the prevention and/or treatment of xenograft recognition and/or rejection,
wherein said prevention and/or treatment comprises inhibiting CD82 expression and/or activity,
wherein said inhibitor is
(i) an anti-CD82 antibody against the CD82 polypeptide as defined in claim 2 or 3,
(ii) small molecule inhibitor(s) of the CD82 expression.

5. The CD82 inhibitor for use according to claim 4, wherein said inhibitor is small molecule inhibitor(s) of the CD82 expression, which are siRNA(s), antisense oligonucleotide(s), transcription and/or translation inhibitor(s).

6. The CD82 inhibitor for use according to claim 4 or 5, wherein the inhibitor is administered to a subject in need thereof, by inhalation, intranasal, intravenous, oral, transdermal, sustained release, controlled release, delayed release, suppository, or sublingual administration.

7. The CD82 inhibitor for use according to any one of claims 4 to 6, wherein the inhibitor is administered to a subject in need thereof in combination with at least one immunosuppressive agent.

8. The CD82 inhibitor for use according to claim 7, wherein the at least one immunosuppressive agent is selected from azathioprene, cyclosporine, glucocorticoid and pharmaceutically acceptable salts thereof.

9. A knockout non-human mammal whose genome comprises a homozygous or heterozygous disruption in a gene encoding a CD82 polypeptide of the mammalian tetraspanin CD82 protein family.

10. A transgenic, non-human mammal, wherein the cells of said non-human mammal fail to express a functional CD82 polypeptide of the mammalian tetraspanin CD82 protein family or wherein the cells of said non-human mammal comprise a coding region of a CD82 polypeptide of the mammalian tetraspanin CD82 protein family under the control of a heterologous promoter active in the cells of said non-human mammal.

11. The knockout or transgenic mammal of claim 9 or 10, wherein said mammal is a pig or a sheep.

12. The knockout or transgenic mammal of any one of claims 9 to 11, wherein the CD82 polypeptide comprises the amino acid sequence of SEQ ID NO. 1 or 2 or an amino acid sequence having at least 80 % sequence identity to SEQ ID NO. 1 or 2;
and/or wherein the CD82 polypeptide is encoded by a nucleotide sequence encoding the amino acid sequence of SEQ ID NO. 1 or 2 or encoding an amino acid sequence having at least 80 % sequence identity to SEQ ID NO. 1 or 2.

13. A cell, a tissue or an organ obtained from the knockout or transgenic mammal of any of claims 9 to 12.

14. The cell(s), tissue(s) and/or organ(s) of claim 13 for use in the prevention and/or treatment of xenograft recognition and/or rejection,
and/or for use in xenotransplantation, such as as xenografts,
wherein the cell(s), tissue(s) and/or organ(s) are administered to a subject in need thereof.

15. A pharmaceutical composition comprising
cell(s), tissue(s) and/or organ(s) obtained from an animal in which the CD82 level,
expression and/or activity is modified or inhibited,
optionally, a pharmaceutical excipient, and
optionally, a pharmaceutical carrier,
wherein said animal in which the CD82 level, expression and/or activity is modified or inhibited is an animal of any of claims 9 to 12,
and/or wherein the cell(s), tissue(s) and/or organ(s) are the cell(s), tissue(s) and/or organ(s) of claim 13.

16. The pharmaceutical composition of claim 15, wherein the carrier, if present, is aqueous.

17. The pharmaceutical composition of any one of claims 15 or 16, furthermore comprising at least one immunosuppressive agent.

18. The pharmaceutical composition of claim 17, wherein the at least one immunosuppressive agent is selected from azathioprene, cyclosporine, glucocorticoid and pharmaceutically acceptable salts thereof.

19. An antibody against the CD82 polypeptide as defined in claim 2 or the pharmaceutical composition of any of claims 15 to 17 for use in the diagnosis, prevention and/or treatment of xenograft recognition and/or rejection.

20. The antibody or the pharmaceutical composition for use according to claim 19, wherein the antibody or the pharmaceutical composition is administered to a subject in need thereof, by inhalation, intranasal, intravenous, oral, transdermal, sustained release, controlled release, delayed release, suppository, or sublingual administration,
and/or wherein the antibody or the pharmaceutical composition is administered to a subject in need thereof in combination with at least one immunosuppressive agent.

21. The antibody or the pharmaceutical composition for use according to claim 20, wherein the at least one immunosuppressive agent is selected from azathioprene, cyclosporine, glucocorticoid and pharmaceutically acceptable salts thereof.

## Patentansprüche

1. Ein CD82 Polypeptid der Säugetier-Tetraspanin CD82 Proteinfamilie zur Verwendung bei der Diagnose von Xenograft-Erkennung und/oder -Abstoßung.

2. Das CD82 Polypeptid zur Verwendung gemäß Anspruch 1, wobei das CD82 Polypeptid die Aminosäuresequenz von SEQ ID NO. 1 oder 2 oder eine Aminosäuresequenz mit mindestens 80 % Sequenzidentität zu SEQ ID NO. 1 oder 2 umfasst,
und/oder wobei das CD82 Polypeptid durch eine Nukleotidesequenz kodiert ist, die die Aminosäuresequenz von SEQ ID NO. 1 oder 2 kodiert oder eine Aminosäuresequenz mit mindestens 80 % Sequenzidentität zu SEQ ID NO. 1 oder 2 kodiert.

3. Das CD82 Polypeptid zur Verwendung gemäß Anspruch 1 oder 2, wobei die Diagnose der Xenograft-Erkennung und/oder -Abstoßung das Bestimmen von CD82 Expressionsspiegeln in Patientenproben umfasst.

4. Ein Inhibitor des CD82 Polypeptids zur Verwendung bei der Prävention und/oder Behandlung von Xenograft-Erkennung und/oder -Abstoßung,
wobei die Prävention und/oder Behandlung das Inhibieren von CD82 Expression und/oder Aktivität umfasst,
wobei der Inhibitor
(i) ein anti-CD82 Antikörper gegen das CD82 Polypeptid, wie definiert in Anspruch 2 oder 3,
(ii) niedermolekulare(r) Inhibitor(en) der CD82 Expression,
ist/sind.

5. Der CD82 Inhibitor zur Verwendung gemäß Anspruch 4, wobei der Inhibitor niedermolekulare(r) Inhibitor(en) der CD82 Expression ist/sind, der/die siRNA(s), Antisense-Oligonukleotid(e), Transkriptions- und/oder Translationsinhibitor(en) ist/sind.

6. Der CD82 Inhibitor zur Verwendung gemäß Anspruch 4 oder 5, wobei der Inhibitor an ein Subjekt, das daran Bedarf hat, verabreicht wird durch Inhalieren, intranasal, intravenös, oral, transdermal, nachhaltige Freisetzung ("sustained release"), kontrollierte Freisetzung, verzögerte Freisetzung, Zäpfchen oder sublinguale Administration.

7. Der CD82 Inhibitor zur Verwendung gemäß einem der Ansprüche 4 bis 6, wobei der Inhibitor an ein Subjekt, das daran Bedarf hat, in Kombination mit mindestens einem immunosuppressiven Mittel verabreicht wird.

8. Der CD82 Inhibitor zur Verwendung gemäß Anspruch 7, wobei das mindestens eine immunosuppressive Mittel ausgewählt ist aus Azathiopren, Cyclosporin, Glucokortikoid und pharmazeutisch akzeptablen Salzen davon.

9. Ein nicht-humanes Knockout-Säugetier, dessen Genom eine homozygote oder heterozygote Disruption in einem Gen umfasst, das ein CD82 Polypeptid der Säugetier-Tetraspanin CD82 Proteinfamilie kodiert.

10. Ein nicht-humanes transgenes Säugetier, wobei die Zellen des nicht-humanen Säugetieres nicht in der Lage sind, ein funktionales CD82 Polypeptid der Säugetier-Tetraspanin CD82 Proteinfamilie zu exprimieren,
oder wobei die Zellen des nicht-humanen Säugetieres eine kodierende Region eines CD82 Polypeptids der Säugetier-Tetraspanin CD82 Proteinfamilie unter der Kontrolle eines heterologen Promoters umfassen, der in den Zellen des nicht-humanen Säugetiers aktiv ist.

11. Das Knockout- oder transgene Säugetier gemäß Anspruch 9 oder 10, wobei das Säugetier ein Schwein oder ein Schaf ist.

12. Das knockout oder transgene Säugetier gemäß einem der Ansprüche 9 bis 11, wobei das CD82 Polypeptid die Aminosäuresequenz von SEQ ID NO. 1 oder 2 oder eine Aminosäuresequenz mit mindestens 80 % Sequenzidentität zu SEQ ID NO. 1 oder 2 umfasst; und/oder wobei das CD82 Polypeptid durch eine Nukleotidesequenz kodiert ist, die die Aminosäuresequenz von SEQ ID NO. 1 oder 2 kodiert oder eine Aminosäuresequenz mit mindestens 80 % Sequenzidentität zu SEQ ID NO. 1 oder 2 kodiert.

13. Eine Zelle, ein Gewebe oder ein Organ erhalten von dem Knockout- oder transgenen Säugetier gemäß einem der Ansprüche 9 bis 12.

14. Die Zelle(n), Gewebe und/oder Organ(e) gemäß Anspruch 13 zur Verwendung bei der Prävention und/oder Behandlung von Xenograft-Erkennung und/oder -Abstoßung und/oder zur Verwendung in der Xenotransplantation, beispielsweise als Xenografts,
wobei die Zelle(n), Gewebe und/oder Organ(e) einem Subjekt, das daran Bedarf hat, verabreicht wird/werden.

15. Eine pharmazeutische Zusammensetzung, umfassend
Zelle(n), Gewebe und/oder Organ(e) erhalten von einem Tier, in welchem der/die CD82 Spiegel, Expression und/oder Aktivität modifiziert oder inhibiert ist,
optional, einen pharmazeutischen Hilfsstoff, und
optional, einen pharmazeutischen Träger,
wobei das Tier, in welchem die CD82 Spiegel, Expression und/oder Aktivität modifiziert oder inhibiert ist, ein Tier gemäß einem der Ansprüche 9 bis 12 ist,
und/oder wobei die Zelle(n), Gewebe und/oder Organ(e) die Zelle(n), Gewebe und/oder Organ(e) gemäß Anspruch 13 sind.

16. Die pharmazeutische Zusammensetzung gemäß Anspruch 15, wobei der Träger, wenn anwesend, wässrig ist.

17. Die pharmazeutische Zusammensetzung gemäß einem der Ansprüche 15 oder 16, weiterhin umfassend mindestens ein immunosuppressives Mittel.

18. Die pharmazeutische Zusammensetzung gemäß Anspruch 17, wobei das mindestens eine immunosuppressive Mittel ausgewählt ist aus Azathiopren, Cyclosporin, Glucokortikoid und pharmazeutisch akzeptablen Salzen davon.

19. Ein Antikörper gegen das CD82 Polypeptid, wie in Anspruch 2 definiert, oder die pharmazeutische Zusammensetzung gemäß einem der Ansprüche 15 bis 17 zur Verwendung bei der Diagnose, Prävention und/oder Behandlung von Xenograft-Erkennung und/oder - Abstoßung.

20. Der Antikörper oder die pharmazeutische Zusammensetzung zur Verwendung gemäß Anspruch 19, wobei der Antikörper oder die pharmazeutische Zusammensetzung an ein Subjekt, das daran Bedarf hat, verabreicht wird durch Inhalieren, intranasal, intravenös, oral, transdermal, nachhaltige Freisetzung ("sustained release"), kontrollierte Freisetzung, verzögerte Freisetzung, Zäpfchen oder sublinguale Administration,
und/oder wobei der Antikörper oder die pharmazeutische Zusammensetzung an ein Subjekt, das daran Bedarf hat, in Kombination mit mindestens einem immunosuppressiven Mittel verabreicht wird.

21. Der Antikörper oder die pharmazeutische Zusammensetzung zur Verwendung gemäß Anspruch 20, wobei das mindestens eine immunosuppressive Mittel ausgewählt ist aus Azathiopren, Cyclosporin, Glucokortikoid und pharmazeutisch akzeptablen Salzen davon.

## Revendications

1. Polypeptide CD82 de la famille des protéines tétraspanines CD82 de mammifère, pour son utilisation dans le diagnostic de la reconnaissance et/ou du rejet d'une xénogreffe.

2. Polypeptide CD82 pour son utilisation selon la revendication 1, dans lequel le polypeptide CD82 comprend la séquence d'acides aminés de SEQ ID NO. 1 ou 2 ou une séquence d'acides aminés dont l'identité de séquence par rapport à SEQ ID NO. 1 ou 2 est au moins de 80 %,
et/ou dans lequel le polypeptide CD82 est codé par une séquence nucléotidique codant la séquence d'acides aminés de SEQ ID NO. 1 ou 2 ou codant une séquence d'acides aminés dont l'identité de séquence par rapport à SEQ ID NO. 1 ou 2 est au moins de 80 %.

3. Polypeptide CD82 pour son utilisation selon la revendication 1 ou 2, dans lequel le diagnostic de ladite reconnaissance et/ou dudit rejet d'une xénogreffe comprend la détermination des niveaux d'expression de CD82 dans un échantillon de patient.

4. Inhibiteur du polypeptide CD82 pour son utilisation dans la prévention et/ou le traitement de la reconnaissance et/ou du rejet d'une xénogreffe,
dans lequel ladite prévention et/ou ledit traitement comprennent l'inhibition de l'expression et/ou de l'activité de CD82,
dans lequel ledit inhibiteur est
(i) un anticorps anti- CD82 dirigé contre le polypeptide CD82 tel que défini dans la revendication 2 ou 3,
(ii) un ou des inhibiteurs à petite molécule de l'expression de CD82.

5. Inhibiteur de CD82 pour son utilisation selon la revendication 4, dans lequel ledit inhibiteur est un ou des inhibiteurs à petite molécule de l'expression de CD82, lequel/lesquels est/sont un/des ARNsi, un/des oligonucléotide(s) antisens, un/des inhibiteur(s) de transcription et/ou de traduction.

6. Inhibiteur de CD82 pour son utilisation selon la revendication 4 ou 5, dans lequel l'inhibiteur est administré à un sujet en ayant besoin, par administration par inhalation, intranasale, intraveineuse, orale, transdermique, à libération prolongée, à libération contrôlée, à libération retardée, par suppositoire ou sublinguale.

7. Inhibiteur de CD82 pour son utilisation selon l'une quelconque des revendications 4 à 6, dans lequel l'inhibiteur est administré à un sujet en ayant besoin en combinaison avec au moins un agent immunosuppresseur.

8. Inhibiteur de CD82 pour son utilisation selon la revendication 7, dans lequel le au moins un agent immunosuppresseur est choisi parmi l'azathioprène, la cyclosporine, un glucocorticoïde et leurs sels pharmaceutiquement acceptables.

9. Mammifère non humain à gène inactivé, dont le génome comprend une disruption homozygote ou hétérozygote dans un gène codant un polypeptide CD82 de la famille des protéines tétraspanines CD82 de mammifère.

10. Mammifère transgénique non humain, dans lequel les cellules dudit mammifère non humain sont incapables d'exprimer un polypeptide CD82 fonctionnel de la famille des protéines tétraspanines CD82 de mammifère ou dans lequel les cellules dudit mammifère non humain comprennent une région codante d'un polypeptide CD82 de la famille des protéines tétraspanines CD82 de mammifère sous la régulation d'un promoteur hétérologue actifs dans les cellules dudit mammifère non humain.

11. Mammifère transgénique ou à gène inactivé selon la revendication 9 ou 10, dans lequel ledit mammifère est un cochon ou un mouton.

12. Mammifère transgénique ou à gène inactivé selon l'une quelconque des revendications 9 à 11, dans lequel le polypeptide CD82 comprend la séquence d'acides aminés de SEQ ID NO. 1 ou 2 ou une séquence d'acides aminés dont l'identité de séquence par rapport à SEQ ID NO. 1 ou 2 est au moins de 80 %,
et/ou dans lequel le polypeptide CD82 est codé par une séquence nucléotidique codant la séquence d'acides aminés de SEQ ID NO. 1 ou 2 ou codant une séquence d'acides aminés dont l'identité de séquence par rapport à SEQ ID NO. 1 ou 2 est au moins de 80 %.

13. Cellule, tissu ou organe obtenu à partir d'un mammifère transgénique ou à gène inactivé selon l'une quelconque des revendications 9 à 12.

14. Cellule(s), tissu(s) et/ou organe (s) selon la revendication 13 pour son utilisation dans la prévention et/ou le traitement de la reconnaissance et/ou du rejet d'une xénogreffe,
et/ou pour son utilisation dans une xénotransplantation, par exemple comme xénogreffes,
dans lequel la/les cellule(s), le/les tissu(s) et/ou l'organe ou les organes est/sont administré(e)(s) à un sujet en ayant besoin.

15. Composition pharmaceutique, comprenant
une/des cellule(s), un/des tissu(s) et/ou un organe ou des organes obtenu(e)(s) à partir d'un animal dans lequel le taux, l'expression et/ou l'activité de CD82 sont modifiés ou inhibés,
facultativement, un excipient pharmaceutique, et
facultativement, un support pharmaceutique,
dans laquelle ledit animal dans lequel le taux, l'expression et/ou l'activité de CD82 sont modifiés ou inhibés est un animal selon l'une quelconque des revendications 9 à 12,
et/ou dans laquelle la/les cellule(s), le/les tissu(s) et/ou l'organe ou les organes est/sont la/les cellule(s), le/les tissu(s) et/ou l'organe ou les organes selon la revendication 13.

16. Composition pharmaceutique selon la revendication 15, dans laquelle le support, s'il est présent, est aqueux.

17. Composition pharmaceutique selon l'une quelconque des revendications 15 ou 16, comprenant en outre au moins un agent immunosuppresseur.

18. Composition pharmaceutique selon la revendication 17, dans laquelle le au moins un agent immunosuppresseur est choisi parmi l'azathioprène, la cyclosporine, un glucocorticoïde et leurs sels pharmaceutiquement acceptables.

19. Anticorps dirigé contre le polypeptide CD82 tel que défini dans la revendication 2 ou composition pharmaceutique selon l'une quelconque des revendications 15 à 17, pour son utilisation dans le diagnostic, la prévention et/ou le traitement de la reconnaissance et/ou du rejet d'une xénogreffe.

20. Anticorps ou composition pharmaceutique pour son utilisation selon la revendication 19, dans lequel l'anticorps ou la composition pharmaceutique est administré à un sujet en ayant besoin, par administration par inhalation, intranasale, intraveineuse, orale, transdermique, à libération prolongée, à libération contrôlée, à libération retardée, par suppositoire ou sublinguale,
et/ou dans lequel l'anticorps ou la composition pharmaceutique est administré à un sujet en ayant besoin en combinaison avec au moins un agent immunosuppresseur.

21. Anticorps ou composition pharmaceutique pour son utilisation selon la revendication 20, dans lequel le au moins un agent immunosuppresseur est choisi parmi l'azathioprène, la cyclosporine, un glucocorticoïde et leurs sels pharmaceutiquement acceptables.


### SEQUENCE LISTING

<110> King Faisal Specialist Hospital & Research Centre
<120> Tetraspanin CD82 as a diagnostic and/or therapeutic module for xenograft recognition and/or rejection
<130> K30901EP
<160> 12
<170> PatentIn version 3.5
<210> 1
   <211> 267
   <212> PRT
   <213> Homo sapiens
<400> 1
   Met Gly Ser Ala Cys Ile Lys Val Thr Lys Tyr Phe Leu Phe Leu Phe
   1               5                   10                  15
   Asn Leu Ile Phe Phe Ile Leu Gly Ala Val Ile Leu Gly Phe Gly Val
               20                  25                  30
   Trp Ile Leu Ala Asp Lys Ser Ser Phe Ile Ser Val Leu Gln Thr Ser
           35                  40                  45
   Ser Ser Ser Leu Arg Met Gly Ala Tyr Val Phe Ile Gly Val Gly Ala
       50                  55                  60
   Val Thr Met Leu Met Gly Phe Leu Gly Cys Ile Gly Ala Val Asn Glu
   65                  70                  75                  80
   Val Arg Cys Leu Leu Gly Leu Tyr Phe Ala Phe Leu Leu Leu Ile Leu
                   85                  90                  95
   Ile Ala Gln Val Thr Ala Gly Ala Leu Phe Tyr Phe Asn Met Gly Lys
               100                 105                 110
   Leu Lys Gln Glu Met Gly Gly Ile Val Thr Glu Leu Ile Arg Asp Tyr
           115                 120                 125
   Asn Ser Ser Arg Glu Asp Ser Leu Gln Asp Ala Trp Asp Tyr Val Gln
       130                 135                 140
   Ala Gln Val Lys Cys Cys Gly Trp Val Ser Phe Tyr Asn Trp Thr Asp
   145                 150                 155                 160
   Asn Ala Glu Leu Met Asn Arg Pro Glu Val Thr Tyr Pro Cys Ser Cys
                   165                 170                 175
   Glu Val Lys Gly Glu Glu Asp Asn Ser Leu Ser Val Arg Lys Gly Phe
   180 185 190
   Cys Glu Ala Pro Gly Asn Arg Thr Gln Ser Gly Asn His Pro Glu Asp
   195 200 205
   Trp Pro Val Tyr Gln Glu Gly Cys Met Glu Lys Val Gln Ala Trp Leu
   210 215 220
   Gln Glu Asn Leu Gly Ile Ile Leu Gly Val Gly Val Gly Val Ala Ile
   225 230 235 240
   Ile Glu Leu Leu Gly Met Val Leu Ser Ile Cys Leu Cys Arg His Val
   245 250 255
   His Ser Glu Asp Tyr Ser Lys Val Pro Lys Tyr
   260 265
<210> 2
   <211> 242
   <212> PRT
   <213> Homo sapiens
<400> 2
   Met Gly Ser Ala Cys Ile Lys Val Thr Lys Tyr Phe Leu Phe Leu Phe
   1               5                   10                  15
   Asn Leu Ile Phe Phe Ile Leu Gly Ala Val Ile Leu Gly Phe Gly Val
               20                  25                  30
   Trp Ile Leu Ala Asp Lys Ser Ser Phe Ile Ser Val Leu Gln Thr Ser
           35                  40                  45
   Ser Ser Ser Leu Arg Met Gly Ala Tyr Val Phe Ile Gly Val Gly Ala
       50                  55                  60
   Val Thr Met Leu Met Gly Phe Leu Gly Cys Ile Gly Ala Val Asn Glu
   65                  70                  75                  80
   Val Arg Cys Leu Leu Gly Leu Leu Lys Gln Glu Met Gly Gly Ile Val
                   85                  90                  95
   Thr Glu Leu Ile Arg Asp Tyr Asn Ser Ser Arg Glu Asp Ser Leu Gln
               100                 105                 110
   Asp Ala Trp Asp Tyr Val Gln Ala Gln Val Lys Cys Cys Gly Trp Val
           115                 120                 125
   Ser Phe Tyr Asn Trp Thr Asp Asn Ala Glu Leu Met Asn Arg Pro Glu
       130                 135                 140
   Val Thr Tyr Pro Cys Ser Cys Glu Val Lys Gly Glu Glu Asp Asn Ser
   145                 150                 155                 160
   Leu Ser Val Arg Lys Gly Phe Cys Glu Ala Pro Gly Asn Arg Thr Gln
                   165                 170                 175
   Ser Gly Asn His Pro Glu Asp Trp Pro Val Tyr Gln Glu Gly Cys Met
               180                 185                 190
   Glu Lys Val Gln Ala Trp Leu Gln Glu Asn Leu Gly Ile Ile Leu Gly
           195                 200                 205
   Val Gly Val Gly Val Ala Ile Ile Glu Leu Leu Gly Met Val Leu Ser
       210                 215                 220
   Ile Cys Leu Cys Arg His Val His Ser Glu Asp Tyr Ser Lys Val Pro
   225                 230                 235                 240
   Lys Tyr
<210> 3
   <211> 20
   <212> DNA
   <213> Artificial Sequence
<220>
   <223> Primer
<400> 3
   ggtcctgtcc atctgcttgt 20
<210> 4
   <211> 20
   <212> DNA
   <213> Artificial Sequence
<220>
   <223> Primer
<400> 4
   ccagaaagcc ccctactttc 20
<210> 5
   <211> 20
   <212> DNA
   <213> Artificial Sequence
<220>
   <223> Primer
<400> 5
   gatggtcctg tccatctgct 20
<210> 6
   <211> 20
   <212> DNA
   <213> Artificial Sequence
<220>
   <223> Primer
<400> 6
   ccagaaagcc ccctactttc 20
<210> 7
   <211> 19
   <212> DNA
   <213> Artificial Sequence
<220>
   <223> Primer
<400> 7
   ggtgaaggtc ggagtcaac 19
<210> 8
   <211> 20
   <212> DNA
   <213> Artificial Sequence
<220>
   <223> Primer
<400> 8
   atgggtggaa tcatattgga 20
<210> 9
   <211> 20
   <212> DNA
   <213> Artificial Sequence
<220>
   <223> Primer
<400> 9
   cagtggtatg ggaaggcact 20
<210> 10
   <211> 20
   <212> DNA
   <213> Artificial Sequence
<220>
   <223> Primer
<400> 10
   agatgacttt gtggccaacc 20
<210> 11
   <211> 20
   <212> DNA
   <213> Artificial Sequence
<220>
   <223> Primer
<400> 11
   gtcggttgtg gatctgacct 20
<210> 12
   <211> 20
   <212> DNA
   <213> Artificial Sequence
<220>
   <223> Primer
<400> 12
   agcttgacga agtggtcgtt 20
7
1